# EUROPEAN PATENT SPECIFICATION

(11) **EP 2 814 526 B1**
(45) Date of publication and mention of the grant of the patent: **02.11.2016**
(21) Application number: 13706876.3
(22) Date of filing: 15.02.2013
(51) Int. Cl.: A61L 27/36, A61L 27/54

(54) **COMPOSITIONS AND IMPROVED SOFT TISSUE REPLACEMENT METHODS**
ZUSAMMENSETZUNGEN UND VERBESSERTE VERFAHREN FÜR DEN ERSATZ VON WEICHGEWEBE
COMPOSITIONS ET PROCÉDÉS PERFECTIONNÉS DE REMPLACEMENT DE TISSU MOU

(30) Priority: 16.02.2012 US 201213398674
(43) Date of publication of application: 24.12.2014
(73) Proprietor: ALLERGAN, INC., Irvine, CA 92612 (US)
(72) Inventor: VAN EPPS, Dennis, E., Goleta, CA 93117 (US); JIANG, Guang-Liang, Irvine, CA 92620 (US); COLLETTE, Adam, L., Westminster, MA 01473 (US); HORAN, Rebecca, L., Arlington, MA 02474 (US); CHEN, Jing, Song, Virginia Beach, VA 23454 (US); ALTMAN, Gregory, H., Arlington, MA 02474 (US); IM, Wha-Bin, Irvine, CA 92612 (US)
(74) Representative: Hoffmann Eitle
(86) International application number: PCT/US2013/026252
(87) International publication number: WO 2013/123274

(56) References cited:
- WO-A2-2010/123945
- WO-A2-2012/112757
- US-A1- 2005 025 755
- CHENG TONG ET AL: "Prostaglandin E2 induces vascular endothelial growth factor and basic fibroblast growth factor mRNA expression in cultured rat Müller cells", INVESTIGATIVE OPHTHALMOLOGY, C.V. MOSBY, ST. LOUIS, US, vol. 39, no. 3, 1 March 1998 (1998-03-01), pages 581-591, XP002659506, ISSN: 0020-9988
- KOJI TAKEUCHI ET AL: "Endogenous prostaglandin E2 accelerates healing of indomethacin-induced small intestinal lesions through upregulation of vascular endothelial growth factor expression by activation of EP4 receptors", JOURNAL OF GASTROENTEROLOGY AND HEPATOLOGY, vol. 25, 1 May 2010 (2010-05-01), pages S67-S74, XP55007377, ISSN: 0815-9319, DOI: 10.1111/j.1440-1746.2010.06222.x

## Description

### BACKGROUND

Soft tissue replacement methods are commonly used for a wide variety of clinical and cosmetic purposes. One use involves reconstructive applications that rebuild and restore a body part or structure to correct deformities from congenital anomalies, trauma, cancer, infections, disease, or medication side effects. The replacement tissue serves to support surrounding tissue and to maintain the normal appearance of the body. The restoration of this normal appearance has an extremely beneficial psychological effect on post-operative patients, alleviating much of the shock and depression that often follows extensive surgical procedures. Another use involves augmentation applications that alter a body part or structure usually to improve its cosmetic or aesthetic appearance. Augmentation of the appearance also has beneficial psychological effects that improve self-esteem, well-being, and confidence of an individual. A third use involves structural applications that provide support to a body part or structure to improve its function, thereby alleviating a symptom associated with a disorder involving soft tissue loss. Examples of such disorders include, without limitation, stress urinary incontinence, fecal incontinence, vocal cord paralysis, vocal atrophy, vocal implantation, intubation trauma, post-hemilaryngectomy defects, irradiation damage, lumbar disc repair, and plantar footpad repair.

Soft tissue replacement methods currently rely on two general approaches: 1) implantation of artificial or alloplastic fillers like soft tissue implants, and injectable polymers and hydrogels; and 2) transplantation of tissue like tissue flaps and autologous tissue transfers. A drawback to the use of artificial or alloplastic fillers it that these inorganic materials lack any metabolic activity, do not become physiologically incorporated into the body, and as such, surrounding tissue and blood supply does not develop within the implanted material. In addition, artificial or alloplastic fillers risk migration and/or extrusion from the implant site. Furthermore, many of these fillers produce only temporary effects because the body rapidly reabsorbs them. Fillers providing long-term effects frequently induce a foreign body response resulting in formation of an avascular, fibrous capsule around the filler, which limits performance, distorts the aesthetic appearance of the surrounding area, and can cause pain to the individual. Furthermore, long-term fillers, like soft tissue implants, do not remodel with the aging tissue resulting in a material that may not be aesthetically acceptable as the patient's tissues undergo the normal physiologic changes associated with aging.

Although the use of transplanted tissue avoids the problems associated with artificial or alloplastic fillers, drawbacks are also associated with these procedures. Examples of fibroin based fillers with adipose tissue transplants are shown in WO2010/123945 A2. In these procedures, loss of transplanted tissue volume over time as a result of its resorption by the body is a major problem, see US2005/0025755 A1. For example, transplantation of adipose tissue generally results in a loss of 20% to 90% of its volume within the first year. This tissue loss is unpredictable and is a result of poor survival and/or regeneration from progenitor cells in the transplanted tissue due to necrosis and a lack of vascular formation. With respect to adipose tissue, tissue breakdown is associated with traumatic rupture of the cells, avascular necrosis, apoptosis of the adipocytes, inflammation secondary to apoptosis, fibrosis and contraction of the graft, and/or delipidation of the adipocytes with subsequent volume loss. Failed tissue grafts sometime produce stellate and irregular nodules with calcifications. As such, transplanted tissue methods are usually performed two or three times to obtain the desired effect, resulting in massive time and cost.

One of the major underlying causes for tissue breakdown seen in transplanted tissue methods is the lack of a blood supply sufficient to support the transplanted tissue. For example, alleviation of tissue ischemia is critically dependent upon formation of new blood vessels. The growth of new blood vessels and associated vasculature or repair or remodeling of existing blood vessels and associated vasculature provides collateral circulation in and around an ischemic area, improves blood flow, and alleviates the symptoms caused by the ischemia. Thus, compositions and methods that promote new blood vessel formation within a transplanted tissue are needed as this will improve the survival rate of such tissues, thereby achieving reliable long-term survival of grafted tissues.

The present invention provides a composition and soft tissue replacement methods that reduce tissue volume loss by increasing the survival rate of the transplanted tissue. This improved survival rate is achieved by administering a compound that promotes new blood vessel formation, thereby ensuring that a blood supply adequate to support the transplanted tissue is established.

### BRIEF SUMMARY

An aspect of the invention is a composition comprising adipose tissue, a silk fibroin material comprising no more than 1% (w/w) of sericin, and a compound represented by the following formula IX: 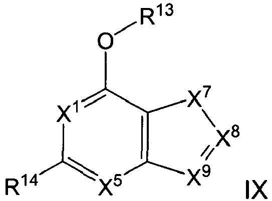 wherein X¹ and X⁵ are independently CH or N; X⁷ is NH, O, or S; X⁸ and X⁹ are independently CH or N; R¹³ is optionally substituted aryl or optionally substituted heteroaryl; and R¹⁴ is C₁₋₆ alkyl, optionally substituted aryl, or optionally substituted heteroaryl.

The above composition can be used to treat a soft tissue condition by promoting the formation of a blood supply sufficient to support transplanted tissue. Non-limiting examples of the soft tissue condition include a breast imperfection, defect, disease and/or disorder, such as, *e.g*., a breast augmentation, a breast reconstruction micromastia, thoracic hypoplasia, Poland's syndrome, defects due to implant complications like capsular contraction and/or rupture; a facial imperfection, defect, disease or disorder, such as, *e.g*., a facial augmentation, a facial reconstruction, Parry-Romberg syndrome, lupus erythematosus profundus, dermal divots, sunken cheeks, thin lips, nasal imperfections or defects, retro-orbital imperfections or defects, a facial fold, line and/or wrinkle like a glabellar line, a nasolabial line, a perioral line, and/or a marionette line, and/or other contour deformities or imperfections of the face; a neck imperfection, defect, disease or disorder; a skin imperfection, defect, disease and/or disorder; other soft tissue imperfections, defects, diseases and/or disorders, such as, *e.g*., an augmentation or a reconstruction of the upper arm, lower arm, hand, shoulder, back, torso including abdomen, buttocks, upper leg, lower leg including calves, foot including plantar fat pad, eye, genitals, or other body part, region or area, or a disease or disorder affecting these body parts, regions or areas; urinary incontinence, fecal incontinence, other forms of incontinence; and gastroesophageal reflux disease (GERD).

A soft tissue condition can be treated by administering an adipose tissue to a site of the soft tissue condition, administering a compound as described above to the site of the soft tissue condition, and administering a silk fibroin material to the site of the soft tissue condition, thereby promoting formation of a blood supply sufficient to support the transplanted tissue. In certain embodiments, the silk fibroin can aid in treating a soft tissue condition by serving one or more functions selected from the group consisting a filling a tissue space, providing a template for tissue reconstruction, providing a template for tissue regeneration, providing a scaffold for cell ingrowth, providing a scaffold for tissue engineering, providing a drug delivery platform, and a combination thereof. Non-limiting examples of a soft tissue condition include those described above.

### DETAILED DESCRIPTION

Insufficient blood vessel formation in a repairing or otherwise metabolically active tissue results in inadequate delivery of oxygen, nutrients, and other substances necessary to establish essential physiological functions to the area and promote wound healing. The formation of blood vessels within a tissue may occur by angiogenesis and/or vasculogenesis. As used herein, the term "angiogenesis" refers to a physiological process involving the growth of new blood vessels from pre-existing vessels and includes sprouting angiogenesis, the formation of new blood vessel by sprouting off existing ones, and splitting angiogenesis (intussusception), the formation of new blood vessel by splitting off existing ones. As used herein, the term "vasculogenesis" refers to a physiological process involving the *de novo* production of new blood-vessels by proliferating endothelial stem cells, and as such, the formation of new blood vessels when there were no pre-existing ones.

Blood vessel formation, whether angiogenesis or vasculogenesis, requires signals from growth factors and other proteins that direct and control the process, such as, *e.g.,* fibroblast growth factors (like FGF-1 and FGF-2), vascular endothelial growth factors (like VEGF-A and VEGF-C), angiopoietins (like Ang-1 and Ang-2), platelet derived growth factor (PDGF), monocyte chemotactic protein-1 (MCP-1) (also known as chemokine (C-C motif) ligand 2 (CCL-2)), transformation growth factor betas (TGF-β1, TGF-β2, TGF-β3, and TGF-β4), vascular cell adhesion molecules (like VCAM-1), matrix metalloproteinases (like MMP-2 and MPP-9), integrins, cadherins, plasminogen activators, plasminogen activator inhibitors, and ephrin.

Of the factors listed above, two of the more critical factors for new blood vessel formation are FGF-2 and VEGF. One of the most important functions of FGF-2 (or bFGF) is the promotion of endothelial cell proliferation and the physical organization of endothelial cells into tube-like structures, thus promoting angiogenesis and vasculogenesis. FGF-2 is a more potent factor in new blood vessel formation than VEGF or PDGF. As well as stimulating blood vessel growth, FGF-2 is an important factor in wound healing. This factor stimulates proliferation of fibroblasts and endothelial cells that give rise to new vessel formation and developing granulation tissue; both increase blood supply and fill up a wound space/cavity early in the wound-healing process.

VEGF is another major contributor to new blood vessel formation. VEGF causes a massive signaling cascade in endothelial cells resulting in the release of many other factors known to be responsible for new blood vessel formation. For example, binding to VEGF receptor-2 (VEGFR-2) starts a tyrosine kinase signaling cascade that stimulates the production of factors that variously stimulate vessel permeability (eNOS, producing NO), proliferation/survival (FGF-2), migration (ICAMs/VCAMs/MMPs) and finally differentiation into mature blood vessels. In addition, *in vitro* studies demonstrated that upon stimulation by VEGF and FGF-2, endothelial cells would proliferate, migrate, and eventually form tube structures resembling capillaries.

The compounds disclosed herein, and compositions comprising such compounds, upregulate both FGF-2 and VEGF (see Example 2). In addition, both incisional and ulcer wound models demonstrate that the upregulation of FGF-2 and VEGF results in an improved wound healing response (see Example 2).

Without wishing to be bound by any particular theory, the compounds, compositions and methods disclosed herein increase the survival rate of transplanted tissue by promoting new blood vessel formation within the transplanted tissue. Administration of a compound, either as a composition with the transplanted tissue or as a prior or subsequent administration to the transplantation procedure, upregulate factors essential to new blood vessel formation, like FGF-2 and VEGF. This upregulation initiates the signaling cascade in endothelial cells responsible for the formation of new blood vessel. This initiation results in the formation of new blood vessels that, in turn, provide oxygen, nutrients, and other substances necessary to establish essential physiological functions to the transplanted tissue. This adequate blood supply supports the establishment, growth and survival of the transplanted tissue. Thus, the compounds, compositions and methods disclosed herein ameliorate or prevent one of the major underlying causes for the tissue breakdown of transplanted tissue, namely the lack of a blood supply sufficient to support the transplanted tissue. In addition, the compounds and compositions disclosed herein are superior to the mere addition of factors critical to new blood vessel formation, like FGF-2 and VEGF, because these small molecules are more stable and less costly then these protein-based therapies. Furthermore, formation of new blood vessels can soften scar tissue, capsular contracture by facilitating the remolding of fibrous tissue into normal soft tissue typical of the affected region.

Aspects of the present specification provide, in part, a composition comprising adipose tissue. As used herein, the term "adipose tissue" is synonymous with "fat" or "fatty tissue" and refers to a loose fibrous connective tissue comprising fat cells (adipocytes) and multiple types of regenerative cells. Adipose tissue may comprise brown and/or white adipose tissue taken from any body site, such as, *e.g.,* subcutaneous, omental/visceral, interscapular, or mediastinal. It may be obtained from any organism having adipose tissue, or the adipose tissue used may be from a primary cell culture or an immortalized cell line.

Adipose tissue may be collected from the same individual who is undergoing the soft tissue replacement procedure (autograft), from a donor individual who is not the same individual as the one undergoing the soft tissue replacement procedure (allograft), or from an animal source (xenograft). As used herein, the term "autotransplantation" refers to the transplantation of organs, tissues, or cells from one part of the body to another part in the same individual, *i.e.,* the donor and recipient are the same individual. Tissue transplanted by such "autologous" procedures is referred to as an autograft or autotransplant. As used herein, the term "allotransplantation" refers to the transplantation of organs, tissues, or cells from a donor to a recipient, where the donor and recipient are different individuals, but of the same species. Tissue transplanted by such "allologous" procedures is referred to as an allograft or allotransplant. As used herein, the term "xenotransplantation" refers to the transplantation of organs, tissues, or cells from a donor to a recipient, where the donor is of a different species as the recipient. Tissue transplanted by such "xenologous" procedures is referred to as a xenograft or xenotransplant.

Adipose tissue can be collected by any procedure that can harvest adipose tissue useful for the compositions and methods disclosed herein, including, without limitation a liposuction (lipoplasty) procedure or a lipectomy procedure. Procedures useful for collecting adipose tissue should minimize the trauma and manipulation associated with adipose tissue removed. Adipose tissue may be harvested from any suitable region, including, without limitation, a mammary region, an abdominal region, a thigh region, a flank region, a gluteal region, a trochanter region, or a gonadal region. Procedures useful for collecting adipose tissue are well known to a person of ordinary skill in the art. The selected procedures may be performed concomitantly with liposculpture.

A liposuction procedure harvests adipose tissue by aspirating the tissue using a cannula. The cannula may be connected to a syringe for manual aspiration or to a power assisted suction device, like an aspirator, adapted to collect the adipose tissue into a vacuum bottle. A liposuction procedure does not maintain an intact blood supply of the harvested tissue. The syringe may be a 10, 20 or 60 mL syringe fitted with a 12 or 14 gauge cannula. Non-limiting examples of liposuction procedures include suction-assisted liposuction (SAL), ultrasound-assisted liposuction (UAL), power-assisted liposuction (PAL), twin-cannula (assisted) liposuction (TCAL or TCL), or external ultrasound-assisted liposuction (XUAL or EUAL), or water-assisted liposuction (WAL). In addition, the liposuction procedures listed above can be used with any of the following procedures that vary the amount of fluid injected during the procedure, such as, *e.g*., dry liposuction, wet liposuction, super-wet liposuction, tumescent liposuction, or laser-assisted liposuction. An autologous soft tissue transfer procedure typically uses adipose tissue collected from a liposuction procedure.

Although the harvested tissue may be used directly to make the disclosed compositions, it is more typically processed to purify and/or enrich for healthy adipocytes and regenerative cells. For example, the harvested adipose tissue may be separated from any debris and/or contaminants such as, *e.g.,* blood, serum, proteases, lipases, lipids and other oils, and/or other bodily fluids; tumescent fluid and/or other materials used in the liposuction procedure; and/or other impurities suctioned during the procedure. Methods useful in separating debris and/or contaminants from adipose tissue useful to make the disclosed compositions, including, without limitation, centrifugation, sedimentation, filtration, and/or absorption. In addition, or alternatively, the harvested adipose tissue may be processed by washing in a physiological buffer like saline to remove any debris and/or contaminants.

A lipectomy procedure harvests adipose tissue by surgical excision from a donor site in a manner that minimizes damage to the blood supply of the tissue using standard surgical operative procedures. This harvested tissue is then implanted into the region needing the soft tissue replacement. A tissue flap or tissue graft procedure typically uses adipose tissue collected from a lipectomy procedure. A tissue flap is a section of living tissue that maintained its blood supply as the tissue is moved from one area of the body to another.

A local flap uses a piece of skin and underlying tissue that lie adjacent to the wound, including adipose tissue. The flap remains attached at one end so that it continues to be nourished by its original blood supply, and is repositioned over the wounded area. A regional flap uses a section of tissue that is attached by a specific blood vessel. When the flap is lifted, it needs only a very narrow attachment to the original site to receive its nourishing blood supply from the tethered artery and vein. A musculocutaneous flap, also called a muscle and skin flap, is used when the area to be covered needs more bulk and a more robust blood supply. Musculocutaneous flaps are often used in breast reconstruction to rebuild a breast after mastectomy. As an example, the transverse rectus abdominus myocutaneous flap (TRAM flap) is a tissue flap procedure that uses muscle, fat and skin from an abdomen to create a new breast mound after a mastectomy. This type of flap remains "tethered" to its original blood supply. In a bone/soft tissue flap, bone, along with the overlying skin, is transferred to the wounded area, carrying its own blood supply.

Typically, a wound that is wide and difficult or impossible to close directly may be treated with a skin graft. A skin graft is a patch of healthy skin that is taken from one area of the body, called the "donor site," and used to cover another area where skin is missing or damaged. There are three basic types of skin grafts. A split-thickness skin graft, commonly used to treat burn wounds, uses only the layers of skin closest to the surface. A full-thickness skin graft might be used to treat a burn wound that is deep and large, or to cover jointed areas where maximum skin elasticity and movement are needed. As its name implies, a full-thickness (all layers) section of skin from the donor site are lifted. A composite graft is used when the wound to be covered needs more underlying support, as with skin cancer on the nose. A composite graft requires lifting all the layers of skin, adipose tissue, and sometimes the underlying cartilage from the donor site.

The amount of adipose tissue collected will typically vary from individual to individual and can depend on a number of factors including, but not limited to, amount of adipose tissue required for the soft tissue replacement method, aesthetic expectations, age, body habitus, coagulation profile, hemodynamic stability, co-morbidities, and physician preference. A liposuction procedure may harvest from about 1 mL to about 1500 mL of adipose tissue. A lipectomy procedure typically harvests about 1 g to about 5,000 g.

Adipose tissue comprises multiple regenerative cells. As used herein, the term "regenerative cell" refers to any cells that cause or contribute to complete or partial regeneration, restoration, or substitution of structure or function of an organ, tissue, or physiologic unit or system to thereby provide a therapeutic, structural or cosmetic benefit. Examples of regenerative cells include stem cells, progenitor cells, and precursor cells. As used herein, the term "stem cell" refers to a multipotent regenerative cell with the potential to differentiate into a variety of other cell types that perform one or more specific functions and has the ability to self-renew. Some of the stem cells disclosed herein may be pluripotent. Exemplary examples of stem cells include, without limitation, adipose-derived stem cells (ASCs; adipose-derived stromal cells), endothelial-derived stem cells (ESCs), hemopoietic stem cells (HSCs), and mesenchyma stem cells (MSCs). As used herein, the term "progenitor cell" refers to an oligopotent regenerative cell with the potential to differentiate into more than one cell type, or a unipotent regenerative cell with the potential to differentiate into only a single cell type, that performs one or more specific functions and has limited or no ability to self-renew. Exemplary examples of progenitor cells include, without limitation, endothelial progenitor cells, keratinocytes, monoblasts, myoblasts, and pericytes. As used herein, the term "precursor cell" refers to a unipotent regenerative cell with the potential to differentiate into one cell type that performs one or more specific functions and may retain extensive proliferative capacity that enables the cells to proliferate under appropriate conditions. Exemplary examples of precursor cells include, without limitation, adipoblast (lipoblast or preadipocytes), de-differentiated adipocytes, angioblasts, endothelial precursor cells, fibroblasts, lymphoblasts, and macrophages.

Harvested adipose tissue can be supplemented with regenerative cells such as, *e.g.,* stem cells, progenitor cells, and precursor cells. Regenerative cells may promote new blood vessel formation, diminish necrosis, and/or promote a supportive microenvironment in the transplanted tissue, thereby improving survivability of the transplanted tissue. Regenerative cells can be obtained form a variety of sources. For example, adipose tissue is rich in regenerative cells that have the ability to restore and reconstruct various soft tissue defects in response to local differentiation clues from the recipient site. As such, a portion of the collected adipose tissue may be further processed in order to purify regenerative cells that can then be added back to the remainder of the harvested adipose tissue in order to enrich this material for these cells. Exemplary methods describing such cell enrichment procedures can be found in, *e.g.*, U.S. Patent Publication 2005/0025755; Yoshimura, et al., Characterization of Freshly Isolated and Cultured Cells Derived form the Fatty and Fluid Portions of liposuction Aspirates, J. Cell. Physiol. 208: 1011-1041 (2006); Yoshimura, et al., Cell-Assisted Lipotransfer for Facial Lipoatrophy: Effects of Clinical Use of Adipose-Derived Stem Cells, Dermatol. Surg. 34: 1178-1185 (2008); and Yoshimura, et al., Cell-Assisted Lipotransfer for Cosmetic Breast Augmentation: Supportive Use of Adipose-Derived Stem/Stromal Cells, Aesth. Plast. Surg. 32: 48-55 (2008).

In addition, harvested adipose tissue can be supplemented with regenerative cells obtained from cell cultures, such as, *e.g.,* primary cell cultures and established cell cultures. For example, a portion of harvested adipose tissue from an individual can be cultured in a manner to produce primary cell cultures enriched for regenerative cells. Alternatively, established cell lines derived from regenerative cells from adipose tissue, or another tissue source, can be cultured, harvested, and added to adipose tissue collected from an individual. Exemplary methods describing such cell culture compositions and procedures can be found in, *e.g*., U.S. Patent Publication 2009/0246182; U.S. Patent Publication 2009/0317367; U.S. Patent Publication 2008/0299213; Rehman, et al., Secretion of Angiogenic and Antiapoptotic Factors by Human Adipose Stromal Cells, Circulation, 109: r52-r58 (2004); and Kilroy, et al., Cytokine Profile of Human Adipose-Derived Stem Cells: Expression of Angiogenic, Hematopoietic, and Pro-Inflammatory Factors, J. Cell. Physiol., 212: 702-709 (2007).

Harvested adipose tissue may be immediately used to make the compositions disclosed herein. Alternatively, harvested adipose tissue, whether unprocessed or processed, may be stored for use at some future date. Harvested tissue is typically stored using a slow freezing method of the tissue to -20 °C, with or without cryopreservatives. Stored adipose tissue can typically be stored for at least 6 months.

Unless otherwise indicated, any reference to a compound herein by structure, name, or any other means, includes pharmaceutically acceptable salts, such as sodium, potassium, and ammonium salts; prodrugs, such as ester prodrugs; alternate solid forms, such as polymorphs, solvates, and hydrates; tautomers; or any other chemical species that may rapidly convert to a compound described herein under conditions in which the compounds are used as described herein.

Unless stereochemistry is unambiguously depicted, any structure or name for a compound used herein may refer to any stereoisomer or any mixture of stereoisomers.

Unless otherwise indicated, when a compound or chemical structural feature such as aryl is referred to as being "optionally substituted," it includes a feature that has no substituents (i.e. "unsubstituted"), or a feature that is "substituted," meaning that the feature has one or more substituents. The term "substituent" has the ordinary meaning known to one of ordinary skill in the art, and includes a moiety that replaces one or more hydrogen atoms attached to a parent compound or structural feature. In some embodiments, the substituent may be an ordinary organic moiety known in the art, which may have a molecular weight (e.g. the sum of the atomic masses of the atoms of the substituent) of 15 g/mol to 50 g/mol, 15 g/mol to 100 g/mol, 15 g/mol to 150 g/mol, 15 g/mol to 200 g/mol, 15 g/mol to 300 g/mol, or 15 g/mol to 500 g/mol. In some embodiments, the substituent comprises: 0-30, 0-20, 0-10, or 0-5 carbon atoms; and 0-30, 0-20, 0-10, or 0-5 heteroatoms independently selected from N, O, S, Si, F, Cl, Br, and I; provided that the substituent comprises at least one atom selected from C, N, O, S, Si, F, Cl, Br, and I. In some embodiments, a substituent may consist of 1, 2, 3, 4, 5, 6, 7, 8, 9, 10, 11, or 12 non-hydrogen atoms and any necessary hydrogen atoms. Some non-hydrogen atoms may include C, N, O, S, Si, F, Cl, Br, I, and P.

Examples of substituents include, but are not limited to, alkyl, alkenyl, alkynyl, heteroalkyl, heteroalkenyl, heteroalkynyl, aryl, heteroaryl, hydroxy, alkoxy, aryloxy, acyl, acyloxy, alkylcarboxylate, carboxylate, thiol, alkylthio, cyano, halo, thiocarbonyl, O-carbamyl, N-carbamyl, O-thiocarbamyl, N-thiocarbamyl, C-amido, N-amido, S-sulfonamido, N-sulfonamido, isocyanato, thiocyanato, isothiocyanato, nitro, silyl, sulfenyl, sulfinyl, sulfonyl, haloalkyl, haloalkoxyl, trihalomethanesulfonyl, trihalomethanesulfonamido, amino, and a combination thereof. The term "combination thereof" in the previous list indicates that substituents may also be a combination of any of the above substituents, wherein a hydrogen atom of one substituent is replaced by another substituent. For example, substituents may be a combination of alkyl and aryl (e.g. CH₂-phenyl, C₂H₄-heteraryl), alkyl and alkoxy (e.g. CH₂OCH₂), alkyl and halo (e.g. C₂H₄Cl, C₃H₆F), or acyl and hydroxyl (e.g. -COCH₂OH).

In some embodiments, a substituent may be R^{A}, F, Cl, CN, OR^{A}, CF₃, NO₂, NR^{A}R^{B}, COR^{A}, CO₂R^{A}, OCORA, NR^{A}COR^{B}, CONR^{A}R^{B},

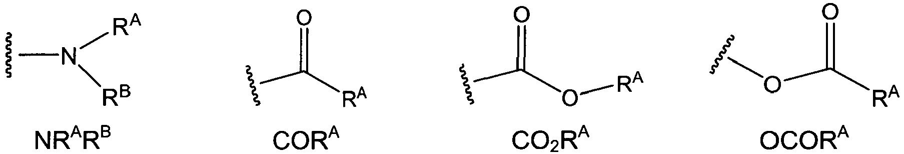

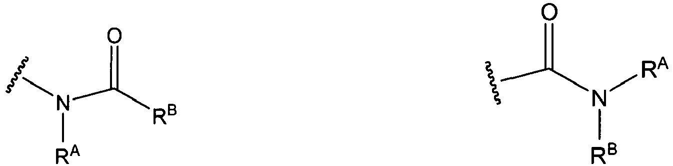

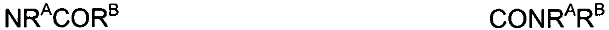

Each R^{A} may independently be H or C₁₋₁₂ alkyl, including linear or branched alkyl having a formula CₐHₐ₊₁, or cycloalkyl having a formula CₐHₐ₋₁, wherein a is 1, 2, 3, 4, 5, 6, 7, 8, 9, 10, 11, or 12, such as linear or branched alkyl of a formula CH₃, C₂H₅, C₃H₇, C₄H₉, C₅H₁₁, C₆H₁₃, C₇H₁₅, C₈H₁₇, C₉H₁₉, C₁₀H_{21,} etc., or cycloalkyl of a formula: C₃H₅, C₄H₇, C₅H₉, C₆H₁₁, C₇H₁₃, C₈H₁₅, C₉H₁₇, C₁₀H₁₉, etc.

Each R^{B} may independently be H or C₁₋₁₂ alkyl, including linear or branched alkyl having a formula CₐHₐ₊₁, or cycloalkyl having a formula CₐHₐ, wherein a is 1, 2, 3, 4, 5, 6, 7, 8, 9, 10, 11, or 12, such as linear or branched alkyl of a formula CH₃, C₂H₅, C₃H₇, C₄H₉, C₅H₁₁, C₆H₁₃, C₈H₁₇, C₇H₁₅, C₉H₁₉, C₁₀H₂₁, etc., or cycloalkyl of a formula: C₃H₅, C₄H₇, C₅H₉, C₆H₁₁, C₇H₁₃, C₈H₁₅, C₉H₁₇, C₁₀H₁₉, etc.

For convenience, the term "molecular weight" is used with respect to a moiety or part of a molecule to indicate the sum of the atomic masses of the atoms in the moiety or part of a molecule, even though it may not be a complete molecule.

Structures associated with some of the chemical names referred to herein are depicted below. These structures may be unsubstituted, as shown below, or a substituent may independently be in any position normally occupied by a hydrogen atom when the structure is unsubstituted. Unless a point of attachment is indicated by 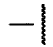 , attachment may occur at any position normally occupied by a hydrogen atom.

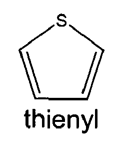

As used herein, the term "hydrocarbyl" has the broadest meaning generally understood in the art, and may include a moiety composed of carbon and hydrogen. Some examples may include alkyl, alkenyl, alkynyl, aryl, and combinations thereof, and may be linear, branched, cyclic, or a combination thereof. Hydrocarbyl may be bonded to any other number of moieties (e.g. be bonded to 1 other group, such as -CH₃ or - CH=CH₂; 2 other groups, such as -phenyl- or -C≡C-; or any number of other groups) that the structure may bear, and in some embodiments, may contain from one to thirty-five carbon atoms. Examples of hydrocarbyl groups include but are not limited to C₁ alkyl, C₂ alkyl, C₂ alkenyl, C₂ alkynyl, C₃ alkyl, C₃ alkenyl, C₃ alkynyl, C₄ alkyl, C₄ alkenyl, C₄ alkynyl, C₅ alkyl, C₅ alkenyl, C₅ alkynyl, C₆ alkyl, C₆ alkenyl, C₆ alkynyl, and phenyl.

As used herein the term "alkyl" has the broadest meaning generally understood in the art, and may include a moiety composed of carbon and hydrogen containing no double or triple bonds. Alkyl may be linear alkyl, branched alkyl, cycloalkyl, or a combination thereof, and in some embodiments, may contain from one to thirty-five carbon atoms. In some embodiments, alkyl may include C₁₋₁₀ linear alkyl, such as methyl (-CH₃), ethyl (-CH₂CH₃), n-propyl (-CH₂CH₂CH₃), n-butyl (-CH₂CH₂CH₂CH₃), n-pentyl (-CH₂CH₂CH₂CH₂CH₃), and n-hexyl (-CH₂CH₂CH₂CH₂CH₂CH₃); C₃₋₁₀ branched alkyl, such as C₃H₇ (e.g. iso-propyl), C₄H₉ (e.g. branched butyl isomers), C₅H₁₁ (e.g. branched pentyl isomers), C₆H₁₃ (e.g. branched hexyl isomers), and C₇H₁₅ (e.g. heptyl isomers); and C₃₋₁₀ cycloalkyl, such as C₃H₅ (e.g. cyclopropyl), C₄H₇ (e.g. cyclobutyl isomers such as cyclobutyl and methylcyclopropyl), C₅H₉ (e.g. cyclopentyl isomers such as cyclopentyl, methylcyclobutyl, and dimethylcyclopropyl,) C₆H₁₁ (e.g. cyclohexyl isomers), and C₇H₁₃ (e.g. cycloheptyl isomers).

As used herein, the term "heterocyclyl" has the broadest meaning generally understood in the art, and may include a heterocyclic ring, or a ring containing one or more heteroatom as ring atoms.

As used herein the term "aryl" has the broadest meaning generally understood in the art, and may include an aromatic ring or aromatic ring system such as phenyl and naphthyl. Any aryl identified herein may be C₆₋₁₀ aryl. The term "heteroaryl" also has the meaning understood by a person of ordinary skill in the art, and may refer to an "aryl" which has one or more heteroatoms in the ring or ring system. Examples of "heteroaryl" may include, but are not limited to, pyridinyl, furyl, thienyl, oxazolyl, thiazolyl, imidazolyl, indolyl, quinolinyl, benzofuranyl, benzothienyl, benzooxazolyl, benzothiazolyl, and benzoimidazolyl. Any heteroaryl identified herein may be C₃₋₉ heteroaryl.

As used herein, the term "haloalkyl" refers to alkyl having one or more halo substituents.

The compound (c) of the invention is represented by the following formula IX: 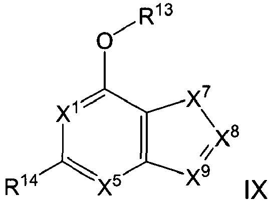 wherein X¹ and X⁵ are independently CH or N; X⁷ is NH, O, or S; X⁸ and X⁹ are independently CH or N; R¹³ is optionally substituted aryl or optionally substituted heteroaryl; and R¹⁴ is C₁₋₆ alkyl, optionally substituted aryl, or optionally substituted heteroaryl.

Methods of preparing the compounds represented by formula IX can be found in, *e.g.,* Linders, et al., *Bicyclic Derivatives as EP4 Agonists,* WO 2008/092860; Linders, et al., *Bicyclic Derivatives as EP4 Agonists,* WO 2008/092861; and Linders, et al., *Bicyclic Derivatives as EP4 Agonists,* WO 2008/092862.

In another embodiment, the compound is represented by the following formula X: 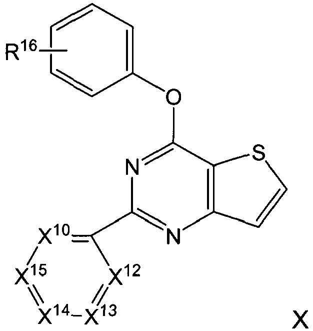 wherein X¹⁰, X¹², X¹³, X¹⁴, and X¹⁵ are each independently CR¹⁹ or N; R¹⁶ is independently H, OH, O, F, Cl, Br, I, C₁₋₆ alkyl, -O-C₁₋₆ alkyl, CN, or C₁₋₆ alkyl-CN; and R¹⁹ is independently H, OH, O, F, Cl, Br, I, C₁₋₁₀ alkyl, -C₁₋₁₀-O-alkyl, or -O-C₄₋₁₀ alkyl-heterocyclyl. The -O-C₄₋₁₀ alkyl-heterocyclyl group can be

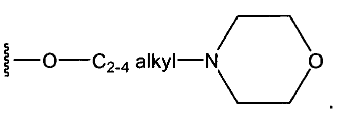

In another embodiment, the compound is represented by the following formula XI: 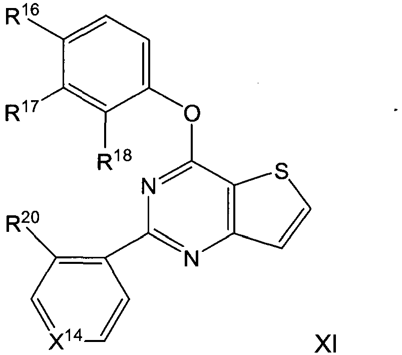 wherein X¹⁴ is CR¹⁹ or N; R¹⁶, R¹⁷, and R¹⁸ are each independently H, OH, O, F, Cl, Br, I, C₁₋₆ alkyl, -O-C₁₋₆ alkyl, CN, or C₁₋₆ alkyl-CN; R¹⁹ is H, OH, O, F, Cl, Br, I, C₁₋₁₀ alkyl,O-C₁₋₁₀ alkyl, or 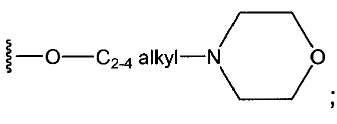 and R²⁰ is H, F, Cl, Br, or I.

In another embodiment, the compound is selected from the following compounds 14, 15, 16, 17, 18, 19, 20, 21, 22, and 23, and pharmaceutically-acceptable salts thereof:

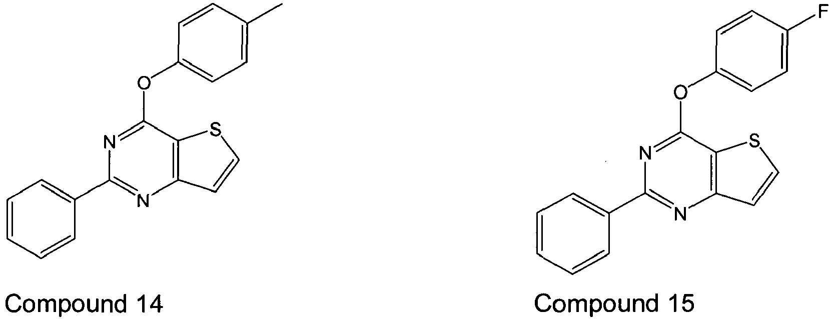

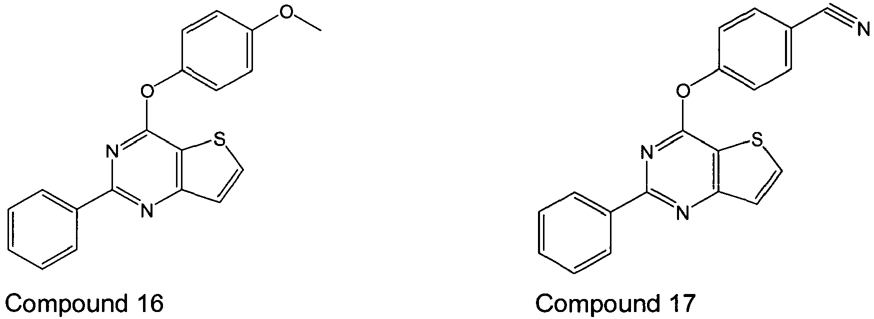

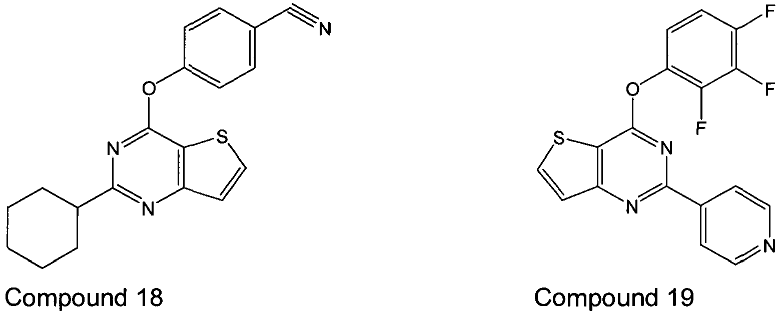

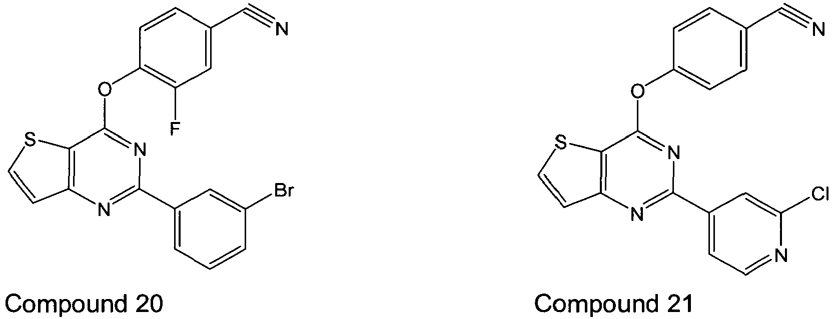

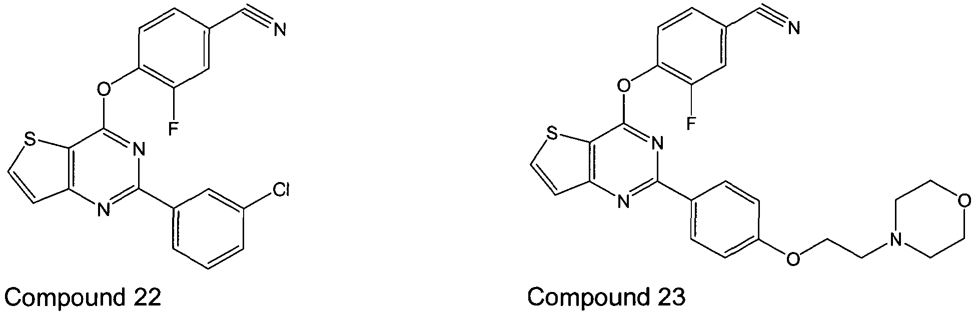

The composition may comprise any number and combination of compounds disclosed herein. For instance, a composition can comprise, *e.g.*, two or more compounds, three or more compounds, four or more compounds, or five or more compounds.

Silk fibroin can serve as, *e.g.,* a filler for tissue space, a template for tissue reconstruction and/or regeneration, a scaffold for cell ingrowth and/or tissue engineering, and/or a drug delivery platform for extended or sustained drug release.

As used herein, the term "depolymerized silk fibroin" is synonymous with "dissolved silk" and "dissolved silk fibroin" and refers to silk fibroin existing primarily as monomers or other lower oligomeric units. Treatment of naturally-occurring fibrous silk with a dissolution agent, such as, *e.g.*, a chaotropic agent results in depolymerized silk fibroin. The depolymerized silk fibroin is an intermediate in the production process and a direct precursor to the silk fibroin material disclosed herein. The depolymerized silk fibroin can be made from raw cocoons, previously degummed silk or any other partially cleaned silk. This may also include material commonly termed as "waste" from the reeling process, i.e. short fragments of raw or degummed silk, the sole precaution being that the silk must be substantially cleaned of sericin prior to making fibroin solution and inducing gel formation. A particular source of raw silk is from common domesticated silkworm *B. mori,* though several other sources of silk may be appropriate. This includes other strains of *Bombycidae* including *Antheraea pernyi, Antheraea yamamai, Antheraea mylitta, Antheraea assama,* and *Philosamia cynthia ricini,* as well as silk producing members of the families *Saturnidae, Thaumetopoeidae,* and silk-producing members of the order *Araneae.* The material may also be obtained from other spider, caterpillar, or recombinant sources. Methods for performing sericin extraction have been described in, *e.g.,* Altman, et al., Sericin Extracted Fabrics, U.S. Patent Publication 2010/0256756; Altman, et al., Sericin Extracted Silkworm Fibroin Fibers, U.S. Patent Publication 2010/0209405; and Altman, et al., Matrix for the production of tissue engineered ligaments, tendons and other tissue, U.S. Patent Publication 2003/0100108.

The silk fibroin of the invention contains no more than 1% (w/w) of sericin. In certain cases, the silk utilized for generation of a silk fibroin material such as, *e.g.,* a hydrogel, a foam, a film, a fiber, a scaffold, a platform, or other silk fibroin material disclosed herein is entirely free of its native sericin content. As used herein, the term "entirely free (i.e. "consisting of" terminology) means that within the detection range of the instrument or process being used, the substance cannot be detected or its presence cannot be confirmed.

Additionally, the possibility exists for deliberately modifying silk fibroin material properties through controlled partial removal of silk sericin. This may function to improve, *e.g.,* hydrophilicity and eventual host acceptance in particular biological settings despite sericin antigenicity.

In aspects of this embodiment, the depolymerized silk fibroin (dissolved silk fibroin) has a concentration of, *e.g.*, about 1 %(w/v), about 2%(w/v), about 3%(w/v), about 4%(w/v), about 5%(w/v), about 6%(w/v), about 7%(w/v), about 8%(w/v), about 9%(w/v), about 10%(w/v), about 12%(w/v), about 15%(w/v), about 18%(w/v), about 20%(w/v), about 25%(w/v), or about 30%(w/v). In other aspects of this embodiment, the depolymerized silk fibroin (dissolved silk fibroin) has a concentration of, *e.g.,* at least 1 %(w/v), at least 2%(w/v), at least 3%(w/v), at least 4%(w/v), at least 5%(w/v), at least 6%(w/v), at least 7%(w/v), at least 8%(w/v), at least 9%(w/v), at least 10%(w/v), at least 12%(w/v), at least 15%(w/v), at least 18%(w/v), at least 20%(w/v), at least 25%(w/v), or at least 30%(w/v). In yet other aspects of this embodiment, the depolymerized silk fibroin (dissolved silk fibroin) has a concentration of, *e.g.,* about 1%(w/v) to about 5% (w/v), about 1%(w/v) to about 10% (w/v), about 1%(w/v) to about 15% (w/v), about 1 %(w/v) to about 20% (w/v), about 1 %(w/v) to about 25% (w/v), about 1 %(w/v) to about 30% (w/v), about 5%(w/v) to about 10% (w/v), about 5%(w/v) to about 15% (w/v), about 5%(w/v) to about 20% (w/v), about 5%(w/v) to about 25% (w/v), about 5%(w/v) to about 30% (w/v), about 10%(w/v) to about 15% (w/v), about 10%(w/v) to about 20% (w/v), about 10%(w/v) to about 25% (w/v), or about 10%(w/v) to about 30% (w/v).

In certain aspects of the disclosure, the depolymerized silk fibroin is utilized to form a silk fibroin material such as, *e.g.,* a hydrogel, a foam, a film, a fiber, a scaffold, a platform, or other silk fibroin material disclosed herein. To form such a silk fibroin material the depolymerized silk fibroin is processed to form polymerized silk fibroin. As used herein, the term "polymerized silk fibroin" is synonymous with "silk fibroin" and refers to silk fibroin existing primarily as a polymer that can be either uncrosslinked or crosslinked.

Silk fibroin hydrogels can be produced by a gelation process using a solution comprising depolymerized silk fibroin. Typically, a gelation enhancer is added to the solution to accelerate polymerization of the silk fibroin and the formation of the hydrogel. Examples of an agent that serves as a gelation enhancer is an alcohol, such as, *e.g*., ethanol, methanol, and isopropanol; glycerol; and acetone. Additionally, various chemical, polypeptide, or peptide additives may be added to influence hydrogel properties and characteristics. Such hydrogel compositions and methods of making are described in, *e.g*., Altman, et al., Silk Fibroin Hydrogels and Uses Thereof, U.S. Patent Application 12/764,038; and Altman, et al., Silk Fibroin Hydrogels and Uses Thereof, U.S. Patent Application 12/764,043.

In certain aspects, a silk fibroin hydrogel may be further processed by pulverizing the hydrogel into particles and optionally mixed with a carrier phase such as, e.g., water or a saline solution to form an injectable or topical substance like a solution, oil, lotion, gel, ointment, cream, slurry, salve, or paste. A hydrogel may be milled to a particle size from about 10 µm to about 1000 µm in diameter. In aspects of this embodiment, a particle size may be about 15 µm to about 30 µm, about 25 µm to about 50 µm, about 45 µm to about 75 µm, about 70 µm to about 100 µm, about 50 µm to about 200 µm, about 150 µm to about 300 µm, about 250 µm to about 400 µm, about 350 µm to about 500 µm, about 450 µm to about 600 µm, about 550 µm to about 700 µm, about 650 µm to about 800 µm, about 750 µm to about 900 µm, or about 850 µm to about 1000 µm. Saline is then added as a carrier phase by first determining the bulk volume of a hydrogel, then vigorously pulverizing the hydrogel into particles while incorporating an appropriate volume of saline to achieve a desired carrier to hydrogel particle ratio. For example, hydrogel milling may be accomplished by means of a forced sieving of bulk hydrogel through a series of stainless steel cloth sieves of decreasing pore sizes.

Silk fibroin sheets or films can be produced by preparing a solution comprising depolymerized silk fibroin and casting the solution. The sheet or film can be contacted with water or water vapor, in the absence of alcohol. The film can then be drawn or stretched mono-axially or biaxially. The stretching of a silk blend film induces molecular alignment of the film and thereby improves the mechanical properties of the film. Such methods are described in, *e.g.*, Kaplin, et al., Silk Biomaterials and Methods of Use Thereof, U.S. Patent 7,674,882; Kaplin, et al., Concentrated Aqueous Silk Fibroin Solution and Use Thereof, U.S. Patent 7,635,755; Kaplin, et al., Silk Fibroin Materials and Use Thereof, US Patent Publication 2006/0273279; and Kaplin, et al., Silk Biomaterials and Methods of Use Thereof, International Patent Publication WO 2004/001103.

Silk fibroin porous materials (like a sponge) may be made from methods known in the art, including, *e.g*., freeze-drying, and/or gas foaming in which water is the solvent or nitrogen or other gas is the blowing agent. Silk porous material may also be made by negative templating or porogen coating. The pore size of a porous material can be controlled, *e.g.,* by adjusting the concentration of silk fibroin and the particle size of the porogen. Contacting the concentrated silk fibroin solution with the porogens is sufficient to induce a conformational change of the amorphous silk to a β-sheet structure that is insoluble in the solution. After formation of the porous material, the excess porogen is then extracted, for example, by immersing in water or other suitable solvent. The resultant porous material can then be dried and used. Such methods are described in, *e.g*., Kaplin, et al., Concentrated Aqueous Silk Fibroin Solution and Use Thereof, U.S. Patent 7,635,755; Ratner and Marshall, Novel Porous Materials, U.S. Patent Publication 2008/0075752; Ma and Chen, Porous Materials having Multi-Sized Geometries, U.S. Patent Publication 2007/0036844; Ma, Reverse Fabrication of Porous Materials, U.S. Patent Publication 2002/0005600; Liu, et al., Porous Materials, Methods of Making and Uses, U.S. Patent Application 61/333,613; Liu, et al., Porous Materials, Methods of Making and Uses, U.S. Patent Application 61/333,120; LeBreton et al., Dermal Fillers Comprising Silk Fibroin Hydrogels Uses Thereof, U.S. Patent Application 12/883,139; and Guang-Liang et al., Drug Delivery Platforms Comprising Silk Fibroin Hydrogels and Uses Thereof, U.S. Patent Application 12/873,563.

Alternatively, silk fibroin porous material may be made using micropatterning techniques. The method comprises contacting a solution comprising depolymerized silk fibroin with a surface of a mold, the mold comprising on at least one surface thereof a three-dimensional negative configuration of a predetermined micropattern to be disposed on and integral with at least one surface of the foam, lyophilizing the solution while in contact with the micropatterned surface of the mold, thereby providing a lyophilized, micropatterned foam, and removing the lyophilized, micropatterned foam from the mold. Foams prepared according to this method comprise a predetermined and designed micropattern on at least one surface, which pattern is effective to facilitate tissue repair, ingrowth or regeneration. Such methods are described in, *e.g.,* Chun, et al., Methods of Making Micropatterned Foams, U.S. Patent 6,423,252.

Silk fibroin fibers may be produced using, for example, wet spinning or electrospinning. Alternatively, as a concentrated solution of silk fibroin has a gel-like consistency, a fiber can be pulled directly from the solution. Electrospinning can be performed by any means known in the art. See, e.g., Zarkoob, et al., Synthetically Spun Silk Nanofibers and a Process for Making the Same, U.S. Patent. 6,110,590. Preferably, a steel capillary tube with a 1.0 mm internal diameter tip is mounted on an adjustable, electrically insulated stand. Preferably, the capillary tube is maintained at a high electric potential and mounted in the parallel plate geometry. The capillary tube is preferably connected to a syringe filled with silk solution. Preferably, a constant volume flow rate is maintained using a syringe pump, set to keep the solution at the tip of the tube without dripping. The electric potential, solution flow rate, and the distance between the capillary tip and the collection screen are adjusted so that a stable jet is obtained. Dry or wet fibers are collected by varying the distance between the capillary tip and the collection screen.

The silk fibroin materials described herein can be further modified after fabrication. For example, the scaffolds can be coated with additives, such as bioactive substances that function as receptors or chemoattractors for a desired population of cells. The coating can be applied through absorption or chemical bonding. See, e.g. Kaplin, et al., Silk Biomaterials and Methods of Use Thereof, U.S. Patent 7,674,882; Kaplin, et al., Concentrated Aqueous Silk Fibroin Solution and Use Thereof, U.S. Patent 7,635,755; Kaplin, et al., Silk Fibroin Materials and Use Thereof, U.S. Patent Publication 2006/0273279; and Kaplin, et al., Silk Biomaterials and Methods of Use Thereof, International Patent Publication WO 2004/001103.

The silk fibroin utilized for generation of a silk fibroin material disclosed herein has been substantially depleted of its native sericin content.

Aspects of the present specification provide, in part, a silk fibroin material disclosed herein that exhibits a complex modulus, an elastic modulus, a viscous modulus and/or a tan δ. The compositions as disclosed herein are viscoelastic in that the composition has an elastic component (solid-like such as, *e.g*., crosslinked silk fibroin material polymers) and a viscous component (liquid-like such as, e.g., uncrosslinked silk fibroin material polymers or a carrier phase) when a force is applied (stress, deformation). The rheological attribute that described this property is the complex modulus (G*), which defines a composition's total resistance to deformation. The complex modulus is a complex number with a real and imaginary part: G*=G'+iG". The absolute value of G* is Abs(G*) = Sqrt(G'²+G"²). The complex modulus can be defined as the sum of the elastic modulus (G') and the viscous modulus (G"). Falcone, et al., Temporary Polysaccharide Dermal Fillers: A Model for Persistence Based on Physical Properties, Dermatol Surg. 35(8): 1238-1243 (2009); Tezel, *supra,* 2008; Kablik, *supra,* 2009; Beasley, *supra,* 2009.

Elastic modulus, or modulus of elasticity, refers to the ability of a silk fibroin material to resists deformation, or, conversely, an object's tendency to be non-permanently deformed when a force is applied to it. Elastic modulus characterizes the firmness of a composition and is also known as the storage modulus because it describes the storage of energy from the motion of the composition. The elastic modulus describes the interaction between elasticity and strength (G' = stress/strain) and, as such, provides a quantitative measurement of a composition's hardness or softness. The elastic modulus of an object is defined as the slope of its stress-strain curve in the elastic deformation region: λ = stress/strain, where λ is the elastic modulus in Pascal's; stress is the force causing the deformation divided by the area to which the force is applied; and strain is the ratio of the change caused by the stress to the original state of the object. Although depending on the speed at which the force is applied, a stiffer composition will have a higher elastic modulus and it will take a greater force to deform the material a given distance, such as, *e.g*., an injection. Specifying how stresses are to be measured, including directions, allows for many types of elastic moduli to be defined. The three primary elastic moduli are tensile modulus, shear modulus, and bulk modulus.

Viscous modulus is also known as the loss modulus because it describes the energy that is lost as viscous dissipation. Tan δ is the ratio of the viscous modulus and the elastic modulus, tan δ = G"/G'. Falcone, *supra,* 2009. For tan δ values disclosed in the present specification, a tan δ is obtained from the dynamic modulus at a frequency of 0.628 rad/s. A lower tan δ corresponds to a stiffer, harder, or more elastic composition.

Thus, in an embodiment, a silk fibroin material disclosed herein exhibits a complex modulus. In aspects of this embodiment, a silk fibroin material exhibits a complex modulus of, *e.g*., about 25 Pa, about 50 Pa, about 75 Pa, about 100 Pa, about 125 Pa, about 150 Pa, about 175 Pa, about 200 Pa, about 250 Pa, about 300 Pa, about 350 Pa, about 400 Pa, about 450 Pa, about 500 Pa, about 550 Pa, about 600 Pa, about 650 Pa, about 700 Pa, about 750 Pa, or about 800 Pa. In other aspects of this embodiment, a silk fibroin material exhibits a complex modulus of, *e.g.,* at most 25 Pa, at most 50 Pa, at most 75 Pa, at most 100 Pa, at most 125 Pa, at most 150 Pa, at most 175 Pa, at most 200 Pa, at most 250 Pa, at most 300 Pa, at most 350 Pa, at most 400 Pa, at most 450 Pa, at most 500 Pa, at most 550 Pa, at most 600 Pa, at most 650 Pa, at most 700 Pa, at most 750 Pa, or at most 800 Pa. In yet other aspects of this embodiment, a silk fibroin material exhibits a complex modulus of, *e.g.*, about 25 Pa to about 150 Pa, about 25 Pa to about 300 Pa, about 25 Pa to about 500 Pa, about 25 Pa to about 800 Pa, about 125 Pa to about 300 Pa, about 125 Pa to about 500 Pa, or about 125 Pa to about 800 Pa.

In another embodiment, a silk fibroin material disclosed herein exhibits an elastic modulus. In aspects of this embodiment, a silk fibroin material exhibits an elastic modulus of, *e.g*., about 25 Pa, about 50 Pa, about 75 Pa, about 100 Pa, about 125 Pa, about 150 Pa, about 175 Pa, about 200 Pa, about 250 Pa, about 300 Pa, about 350 Pa, about 400 Pa, about 450 Pa, about 500 Pa, about 550 Pa, about 600 Pa, about 650 Pa, about 700 Pa, about 750 Pa, about 800 Pa, about 850 Pa, about 900 Pa, about 950 Pa, about 1,000 Pa, about 1,200 Pa, about 1,300 Pa, about 1,400 Pa, about 1,500 Pa, about 1,600 Pa, about 1700 Pa, about 1800 Pa, about 1900 Pa, about 2,000 Pa, about 2,100 Pa, about 2,200 Pa, about 2,300 Pa, about 2,400 Pa, or about 2,500 Pa. In other aspects of this embodiment, a silk fibroin material exhibits an elastic modulus of, *e.g.,* at least 25 Pa, at least 50 Pa, at least 75 Pa, at least 100 Pa, at least 125 Pa, at least 150 Pa, at least 175 Pa, at least 200 Pa, at least 250 Pa, at least 300 Pa, at least 350 Pa, at least 400 Pa, at least 450 Pa, at least 500 Pa, at least 550 Pa, at least 600 Pa, at least 650 Pa, at least 700 Pa, at least 750 Pa, at least 800 Pa, at least 850 Pa, at least 900 Pa, at least 950 Pa, at least 1,000 Pa, at least 1,200 Pa, at least 1,300 Pa, at least 1,400 Pa, at least 1,500 Pa, at least 1,600 Pa, at least 1700 Pa, at least 1800 Pa, at least 1900 Pa, at least 2,000 Pa, at least 2,100 Pa, at least 2,200 Pa, at least 2,300 Pa, at least 2,400 Pa, or at least 2,500 Pa. In yet other aspects of this embodiment, a silk fibroin material exhibits an elastic modulus of, *e.g.,* at most 25 Pa, at most 50 Pa, at most 75 Pa, at most 100 Pa, at most 125 Pa, at most 150 Pa, at most 175 Pa, at most 200 Pa, at most 250 Pa, at most 300 Pa, at most 350 Pa, at most 400 Pa, at most 450 Pa, at most 500 Pa, at most 550 Pa, at most 600 Pa, at most 650 Pa, at most 700 Pa, at most 750 Pa, at most 800 Pa, at most 850 Pa, at most 900 Pa, at most 950 Pa, at most 1,000 Pa, at most 1,200 Pa, at most 1,300 Pa, at most 1,400 Pa, at most 1,500 Pa, or at most 1,600 Pa. In still other aspects of this embodiment, a silk fibroin material exhibits an elastic modulus of, *e.g.,* about 25 Pa to about 150 Pa, about 25 Pa to about 300 Pa, about 25 Pa to about 500 Pa, about 25 Pa to about 800 Pa, about 125 Pa to about 300 Pa, about 125 Pa to about 500 Pa, about 125 Pa to about 800 Pa, about 500 Pa to about 1,600 Pa, about 600 Pa to about 1,600 Pa, about 700 Pa to about 1,600 Pa, about 800 Pa to about 1,600 Pa, about 900 Pa to about 1,600 Pa, about 1,000 Pa to about 1,600 Pa, about 1,100 Pa to about 1,600 Pa, about 1,200 Pa to about 1,600 Pa, about 500 Pa to about 2,500 Pa, about 1,000 Pa to about 2,500 Pa, about 1,500 Pa to about 2,500 Pa, about 2,000 Pa to about 2,500 Pa, about 1,300 Pa to about 1,600 Pa, about 1,400 Pa to about 1,700 Pa, about 1,500 Pa to about 1,800 Pa, about 1,600 Pa to about 1,900 Pa, about 1,700 Pa to about 2,000 Pa, about 1,800 Pa to about 2,100 Pa, about 1,900 Pa to about 2,200 Pa, about 2,000 Pa to about 2,300 Pa, about 2,100 Pa to about 2,400 Pa, or about 2,200 Pa to about 2,500 Pa.

In another embodiment, a silk fibroin material disclosed herein exhibits a tensile modulus. In aspects of this embodiment, a silk fibroin material exhibits a tensile modulus of, *e.g*., about 1 MPa, about 10 MPa, about 20 MPa, about 30 MPa, about 40 MPa, about 50 MPa, about 60 MPa, about 70 MPa, about 80 MPa, about 90 MPa, about 100 MPa, about 200 MPa, about 300 MPa, about 400 MPa, about 500 MPa, about 750 MPa, about 1 GPa, about 5 GPa, about 10 GPa, about 15 GPa, about 20 GPa, about 25 GPa, or about 30 GPa. In other aspects of this embodiment, a silk fibroin material exhibits a tensile modulus of, *e.g*., at least 1 MPa, at least 10 MPa, at least 20 MPa, at least 30 MPa, at least 40 MPa, at least 50 MPa, at least 60 MPa, at least 70 MPa, at least 80 MPa, at least 90 MPa, at least 100 MPa, at least 200 MPa, at least 300 MPa, at least 400 MPa, at least 500 MPa, at least 750 MPa, at least 1 GPa, at least 5 GPa, at least 10 GPa, at least 15 GPa, at least 20 GPa, at least 25 GPa, or at least 30 GPa In yet other aspects of this embodiment, a silk fibroin material exhibits a tensile modulus of, *e.g*., about 1 MPa to about 30 MPa, about 10 MPa to about 50 MPa, about 25 MPa to about 75 MPa, about 50 MPa to about 100 MPa, about 100 MPa to about 300 MPa, about 200 MPa to about 400 MPa, about 300 MPa to about 500 MPa, about 100 MPa to about 500 MPa, about 250 MPa to about 750 MPa, about 500 MPa to about 1 GPa, about 1 GPa to about 30 GPa, about 10 GPa to about 30 GPa.

In another embodiment, a silk fibroin material disclosed herein exhibits shear modulus. In aspects of this embodiment, a silk fibroin material exhibits a shear modulus of, *e.g*., about 1 MPa, about 10 MPa, about 20 MPa, about 30 MPa, about 40 MPa, about 50 MPa, about 60 MPa, about 70 MPa, about 80 MPa, about 90 MPa, about 100 MPa, about 200 MPa, about 300 MPa, about 400 MPa, about 500 MPa, about 750 MPa, about 1 GPa, about 5 GPa, about 10 GPa, about 15 GPa, about 20 GPa, about 25 GPa, or about 30 GPa. In other aspects of this embodiment, a silk fibroin material exhibits a shear modulus of, *e.g*., at least 1 MPa, at least 10 MPa, at least 20 MPa, at least 30 MPa, at least 40 MPa, at least 50 MPa, at least 60 MPa, at least 70 MPa, at least 80 MPa, at least 90 MPa, at least 100 MPa, at least 200 MPa, at least 300 MPa, at least 400 MPa, at least 500 MPa, at least 750 MPa, at least 1 GPa, at least 5 GPa, at least 10 GPa, at least 15 GPa, at least 20 GPa, at least 25 GPa, or at least 30 GPa In yet other aspects of this embodiment, a silk fibroin material exhibits a shear modulus of, *e.g*., about 1 MPa to about 30 MPa, about 10 MPa to about 50 MPa, about 25 MPa to about 75 MPa, about 50 MPa to about 100 MPa, about 100 MPa to about 300 MPa, about 200 MPa to about 400 MPa, about 300 MPa to about 500 MPa, about 100 MPa to about 500 MPa, about 250 MPa to about 750 MPa, about 500 MPa to about 1 GPa, about 1 GPa to about 30 GPa, about 10 GPa to about 30 GPa.

In another embodiment, a silk fibroin material disclosed herein exhibits a bulk modulus. In aspects of this embodiment, a silk fibroin material exhibits a bulk modulus of, *e.g*., about 5 GPa, about 6 GPa, about 7 GPa, about 8 GPa, about 9 GPa, about 10 GPa, about 15 GPa, about 20 GPa, about 25 GPa, about 30 GPa, about 35 GPa, about 40 GPa, about 45 GPa, about 50 GPa, about 60 GPa, about 70 GPa, about 80 GPa, about 90 GPa, about 100 GPa. In other aspects of this embodiment, a silk fibroin material exhibits a bulk modulus of, *e.g.,* at least 5 GPa, at least 6 GPa, at least 7 GPa, at least 8 GPa, at least 9 GPa, at least 10 GPa, at least 15 GPa, at least 20 GPa, at least 25 GPa, at least 30 GPa, at least 35 GPa, at least 40 GPa, at least 45 GPa, at least 50 GPa, at least 60 GPa, at least 70 GPa, at least 80 GPa, at least 90 GPa, at least 100 GPa. In yet other aspects of this embodiment, a silk fibroin material exhibits a bulk modulus of, *e.g.,* about 5 GPa to about 50 GPa, about 5 GPa to about 100 GPa, about 10 GPa to about 50 GPa, about 10 GPa to about 100 GPa, or about 50 GPa to about 100 GPa.

In another embodiment, a silk fibroin material disclosed herein exhibits a viscous modulus. In aspects of this embodiment, a silk fibroin material exhibits a viscous modulus of, *e*.*g*., about 10 Pa, about 20 Pa, about 30 Pa, about 40 Pa, about 50 Pa, about 60 Pa, about 70 Pa, about 80 Pa, about 90 Pa, about 100 Pa, about 150 Pa, about 200 Pa, about 250 Pa, about 300 Pa, about 350 Pa, about 400 Pa, about 450 Pa, about 500 Pa, about 550 Pa, about 600 Pa, about 650 Pa, or about 700 Pa. In other aspects of this embodiment, a silk fibroin material exhibits a viscous modulus of, *e.g.,* at most 10 Pa, at most 20 Pa, at most 30 Pa, at most 40 Pa, at most 50 Pa, at most 60 Pa, at most 70 Pa, at most 80 Pa, at most 90 Pa, at most 100 Pa, at most 150 Pa, at most 200 Pa, at most 250 Pa, at most 300 Pa, at most 350 Pa, at most 400 Pa, at most 450 Pa, at most 500 Pa, at most 550 Pa, at most 600 Pa, at most 650 Pa, or at most 700 Pa.. In yet other aspects of this embodiment, a silk fibroin material exhibits a viscous modulus of, *e.g.,* about 10 Pa to about 30 Pa, about 10 Pa to about 50 Pa, about 10 Pa to about 100 Pa, about 10 Pa to about 150 Pa, about 70 Pa to about 100 Pa, about 50 Pa to about 350 Pa, about 150 Pa to about 450 Pa, about 250 Pa to about 550 Pa, about 350 Pa to about 700 Pa, about 50 Pa to about 150 Pa, about 100 Pa to about 200 Pa, about 150 Pa to about 250 Pa, about 200 Pa to about 300 Pa, about 250 Pa to about 350 Pa, about 300 Pa to about 400 Pa, about 350 Pa to about 450 Pa, about 400 Pa to about 500 Pa, about 450 Pa to about 550 Pa, about 500 Pa to about 600 Pa, about 550 Pa to about 650 Pa, or about 600 Pa to about 700 Pa.

In another embodiment, a silk fibroin material disclosed herein exhibits a tan δ. In aspects of this embodiment, a silk fibroin material exhibits a tan δ of, *e.g.,* about 0.1, about 0.2, about 0.3, about 0.4, about 0.5, about 0.6, about 0.7, about 0.8, about 0.9, about 1.0, about 1.1, about 1.2, about 1.3, about 1.4, about 1.5, about 1.6, about 1.7, about 1.8, about 1.9, about 2.0, about 2.1, about 2.2, about 2.3, about 2.4, or about 2.5. In other aspects of this embodiment, a silk fibroin material exhibits a tan δ of, e.g., at most 0.1, at most 0.2, at most 0.3, at most 0.4, at most 0.5, at most 0.6, at most 0.7, at most 0.8, at most 0.9, at most 1.0, at most 1.1, at most 1.2, at most 1.3, at most 1.4, at most 1.5, at most 1.6, at most 1.7, at most 1.8, at most 1.9, at most 2.0, at most 2.1, at most 2.2, at most 2.3, at most 2.4, or at most 2.5. In yet other aspects of this embodiment, a silk fibroin material exhibits a tan δ of, *e.g.,* about 0.1 to about 0.3, about 0.3 to about 0.5, about 0.5 to about 0.8, about 1.1 to about 1.4, about 1.4 to about 1.7, about 0.3 to about 0.6, about 0.1 to about 0.5, about 0.5 to about 0.9, about 0.1 to about 0.6, about 0.1 to about 1.0, about 0.5 to about 1.5, about 1.0 to about 2.0, or about 1.5 to about 2.5.

Aspects of the present specification provide, in part, a silk fibroin material disclosed herein having hardness. Hardness refers to various properties of an object in the solid phase that gives it high resistance to various kinds of shape change when force is applied. Hardness is measured using a durometer and is a unitless value that ranges from zero to 100. The ability or inability of a silk fibroin material to be easily compressed will affect its suitability for application in different tissue replacement roles, i.e., mechanical compliance as bone, fat, connective tissue. Hardness will also affect the ability of a silk fibroin material to be effectively comminuted, the reason being that a hard material may be more easily and consistently comminuted. Hardness will also affect extrudability, as a soft material may be more readily able to be slightly compressed during injection to pack with other particles or change shape to pass through a syringe barrel or needle.

In an embodiment, a silk fibroin material disclosed herein exhibits low hardness. In aspects of this embodiment, a silk fibroin material exhibits a hardness of, e.g., about 5, about 10, about 15, about 20, about 25, about 30, or about 35. In other aspects of this embodiment, a silk fibroin material exhibits a hardness of, *e.g.,* at most 5, at most 10, at most 15, at most 20, at most 25, at most 30, or at most 35. In yet other aspects of this embodiment, a silk fibroin material exhibits a hardness of, *e.g.,* about 5 to about 35, about 10 to about 35, about 15 to about 35, about 20 to about 35, or about 25 to about 35, about 5 to about 40, about 10 to about 40, about 15 to about 40, about 20 to about 40, about 25 to about 40, or about 30 to about 40.

In an embodiment, a silk fibroin material disclosed herein exhibits medium hardness. In aspects of this embodiment, a silk fibroin material exhibits a hardness of, *e.g.,* about 40, about 45, about 50, about 55, or about 60. In other aspects of this embodiment, a silk fibroin material exhibits a hardness of, *e.g.,* at least 40, at least 45, at least 50, at least 55, or at least 60. In yet other aspects of this embodiment, a silk fibroin material exhibits a hardness of, *e.g.,* at most 40, at most 45, at most 50, at most 55, or at most 60. In still other aspects of this embodiment, a silk fibroin material exhibits a hardness of, *e.g.,* about 35 to about 60, about 35 to about 55, about 35 to about 50, about 35 to about 45, about 40 to about 60, about 45 to about 60, about 50 to about 60, about 55 to about 60, about 40 to about 65, about 45 to about 65, about 50 to about 65, about 55 to about 65.

In another embodiment, a silk fibroin material disclosed herein exhibits high hardness. In aspects of this embodiment, a silk fibroin material exhibits a hardness of, *e.g.,* about 65, about 70, about 75, about 80, about 85, about 90, about 95, or about 100. In other aspects of this embodiment, a silk fibroin material exhibits a hardness of, *e.g.,* at least 65, at least 70, at least 75, at least 80, at least 85, at least 90, at least 95, or at least 100. In yet other aspects of this embodiment, a silk fibroin material exhibits a hardness of, *e.g.,* about 65 to about 100, about 70 to about 100, about 75 to about 100,about 80 to about 100, about 85 to about 100, about 90 to about 100, about 65 to about 75, about 65 to about 80, about 65 to about 85, about 65 to about 90, about 65 to about 95, about 60 to about 75, about 60 to about 80, about 60 to about 85, about 60 to about 90, or about 60 to about 95.

In an embodiment, a silk fibroin material disclosed herein exhibits high resistant to deformation. In aspects of this embodiment, a silk fibroin material exhibits resistant to deformation of, *e.g.,* about 100%, about 99%, about 98%, about 97%, about 96%, about 95%, about 94%, about 93%, about 92%, about 91%, about 90%, about 89%, about 88%, about 87%, about 86%, or about 85%. In other aspects of this embodiment, a silk fibroin material exhibits resistant to deformation of, *e.g.,* at least 99%, at least 98%, at least 97%, at least 96%, at least 95%, at least 94%, at least 93%, at least 92%, at least 91%, at least 90%, at least 89%, at least 88%, at least 87%, at least 86%, or at least 85%. In yet other aspects of this embodiment, a silk fibroin material exhibits resistant to deformation of, *e.g.,* at most 99%, at most 98%, at most 97%, at most 96%, at most 95%, at most 94%, at most 93%, at most 92%, at most 91%, at most 90%, at most 89%, at most 88%, at most 87%, at most 86%, or at most 85%. In still aspects of this embodiment, a silk fibroin material exhibits resistant to deformation of, *e.g.,* about 85% to about 100%, about 87% to about 100%, about 90% to about 100%, about 93% to about 100%, about 95% to about 100%, or about 97% to about 100%.

A silk fibroin silk material disclosed herein exhibits high tensile strength. Tensile strength has three different definitional points of stress maxima. Yield strength refers to the stress at which material strain changes from elastic deformation to plastic deformation, causing it to deform permanently. Ultimate strength refers to the maximum stress a material can withstand when subjected to tension, compression or shearing. It is the maximum stress on the stress-strain curve. Breaking strength refers to the stress coordinate on the stress-strain curve at the point of rupture, or when the material pulls apart.

In another embodiment, a silk fibroin silk material disclosed herein exhibits high yield strength relative to other polymer classes. In aspects of this embodiment, a silk fibroin material exhibits a yield strength of, *e.g.,* about 0.1 MPa, about 0.5 MPa, about 1 MPa, about 5 MPa, about 10 MPa, about 20 MPa, about 30 MPa, about 40 MPa, about 50 MPa, about 60 MPa, about 70 MPa, about 80 MPa, about 90 MPa, about 100 MPa, about 200 MPa, about 300 MPa, about 400 MPa, about 500 MPa. In other aspects of this embodiment, a silk fibroin material exhibits a yield strength of, *e.g.,* at least 0.1 MPa, at least 0.5 MPa, at least 1 MPa, at least 5 MPa, at least 10 MPa, at least 20 MPa, at least 30 MPa, at least 40 MPa, at least 50 MPa, at least 60 MPa, at least 70 MPa, at least 80 MPa, at least 90 MPa, at least 100 MPa, at least 200 MPa, at least 300 MPa, at least 400 MPa, at least 500 MPa. In yet other aspects of this embodiment, a silk fibroin material exhibits a yield strength of, *e.g.,* at most 1 MPa, at most 5 MPa, at most 10 MPa, at most 20 MPa, at most 30 MPa, at most 40 MPa, at most 50 MPa, at most 60 MPa, at most 70 MPa, at most 80 MPa, at most 90 MPa, at most 100 MPa, at most 200 MPa, at most 300 MPa, at most 400 MPa, at most 500 MPa, at most 600 MPa, at most 700 MPa, at most 800 MPa, at most 900 MPa, at most 1000 MPa, at most 1500 MPa, or at most 2000 MPa. In still other aspects of this embodiment, a silk fibroin material exhibits a yield strength of, *e.g.,* about 1 MPa to about 50 MPa, about 1 MPa to about 60 MPa, about 1 MPa to about 70 MPa, about 1 MPa to about 80 MPa, about 1 MPa to about 90 MPa, about 1 MPa to about 100 MPa, about 10 MPa to about 50 MPa, about 10 MPa to about 60 MPa, about 10 MPa to about 70 MPa, about 10 MPa to about 80 MPa, about 10 MPa to about 90 MPa, about 10 MPa to about 100 MPa, about 10 MPa to about 200 MPa, about 10 MPa to about 300 MPa, or about 100 MPa to about 300 MPa.

In another embodiment, a silk fibroin silk material disclosed herein exhibits high ultimate strength. In aspects of this embodiment, a silk fibroin material exhibits an ultimate strength of, *e.g.,* about 0.1 MPa, about 0.5 MPa, about 1 MPa, about 5 MPa, about 10 MPa, about 20 MPa, about 30 MPa, about 40 MPa, about 50 MPa, about 60 MPa, about 70 MPa, about 80 MPa, about 90 MPa, about 100 MPa, about 200 MPa, about 300 MPa, about 400 MPa, about 500 MPa. In other aspects of this embodiment, a silk fibroin material exhibits an ultimate strength of, *e.g.,* at least 0.1 MPa, at least 0.5 MPa, at least 1 MPa, at least 5 MPa, at least 10 MPa, at least 20 MPa, at least 30 MPa, at least 40 MPa, at least 50 MPa, at least 60 MPa, at least 70 MPa, at least 80 MPa, at least 90 MPa, at least 100 MPa, at least 200 MPa, at least 300 MPa, at least 400 MPa, at least 500 MPa. In yet other aspects of this embodiment, a silk fibroin material exhibits an ultimate strength of, *e.g.,* at most 1 MPa, at most 5 MPa, at most 10 MPa, at most 20 MPa, at most 30 MPa, at most 40 MPa, at most 50 MPa, at most 60 MPa, at most 70 MPa, at most 80 MPa, at most 90 MPa, at most 100 MPa, at most 200 MPa, at most 300 MPa, at most 400 MPa, at most 500 MPa, at most 600 MPa, at most 700 MPa, at most 800 MPa, at most 900 MPa, at most 1000 MPa, at most 1500 MPa, or at most 2000 MPa. In still other aspects of this embodiment, a silk fibroin material exhibits an ultimate strength of, *e.g.,* about 1 MPa to about 50 MPa, about 1 MPa to about 60 MPa, about 1 MPa to about 70 MPa, about 1 MPa to about 80 MPa, about 1 MPa to about 90 MPa, about 1 MPa to about 100 MPa, about 10 MPa to about 50 MPa, about 10 MPa to about 60 MPa, about 10 MPa to about 70 MPa, about 10 MPa to about 80 MPa, about 10 MPa to about 90 MPa, about 10 MPa to about 100 MPa, about 10 MPa to about 200 MPa, about 10 MPa to about 300 MPa, or about 100 MPa to about 300 MPa.

In another embodiment, a silk fibroin silk material disclosed herein exhibits high breaking strength. In aspects of this embodiment, a silk fibroin material exhibits a breaking strength of, *e.g.,* about 0.1 MPa, about 0.5 MPa, about 1 MPa, about 5 MPa, about 10 MPa, about 20 MPa, about 30 MPa, about 40 MPa, about 50 MPa, about 60 MPa, about 70 MPa, about 80 MPa, about 90 MPa, about 100 MPa, about 200 MPa, about 300 MPa, about 400 MPa, about 500 MPa. In other aspects of this embodiment, a silk fibroin material exhibits a breaking strength of, *e.g.,* at least 0.1 MPa, at least 0.5 MPa, at least 1 MPa, at least 5 MPa, at least 10 MPa, at least 20 MPa, at least 30 MPa, at least 40 MPa, at least 50 MPa, at least 60 MPa, at least 70 MPa, at least 80 MPa, at least 90 MPa, at least 100 MPa, at least 200 MPa, at least 300 MPa, at least 400 MPa, at least 500 MPa. In yet other aspects of this embodiment, a silk fibroin material exhibits a breaking strength of, *e.g.,* at most 1 MPa, at most 5 MPa, at most 10 MPa, at most 20 MPa, at most 30 MPa, at most 40 MPa, at most 50 MPa, at most 60 MPa, at most 70 MPa, at most 80 MPa, at most 90 MPa, at most 100 MPa, at most 200 MPa, at most 300 MPa, at most 400 MPa, at most 500 MPa, at most 600 MPa, at most 700 MPa, at most 800 MPa, at most 900 MPa, at most 1000 MPa, at most 1500 MPa, or at most 2000 MPa. In still other aspects of this embodiment, a silk fibroin material exhibits a breaking strength of, *e.g.,* about 1 MPa to about 50 MPa, about 1 MPa to about 60 MPa, about 1 MPa to about 70 MPa, about 1 MPa to about 80 MPa, about 1 MPa to about 90 MPa, about 1 MPa to about 100 MPa, about 10 MPa to about 50 MPa, about 10 MPa to about 60 MPa, about 10 MPa to about 70 MPa, about 10 MPa to about 80 MPa, about 10 MPa to about 90 MPa, about 10 MPa to about 100 MPa, about 10 MPa to about 200 MPa, about 10 MPa to about 300 MPa, or about 100 MPa to about 300 MPa.

Aspects of the present specification provide, in part, a silk fibroin material disclosed herein having a transparency and/or translucency. Transparency (also called pellucidity or diaphaneity) is the physical property of allowing light to pass through a material, whereas translucency (also called translucence or translucidity) only allows light to pass through diffusely. The opposite property is opacity. Transparent materials are clear, while translucent ones cannot be seen through clearly. The silk fibroin material disclosed herein may optionally exhibit optical properties such as transparency and translucency. In certain cases, e.g., superficial line filling, it would be an advantage to have an opaque silk fibroin material. In other cases such as development of a lens or a "humor" for filling the eye, it would be an advantage to have a translucent silk fibroin material. These properties could be modified by affecting the structural distribution of the silk fibroin material. Factors used to control a silk fibroin material's optical properties include, without limitation, polymer concentration, gel crystallinity, and material homogeneity.

When light encounters a material, it can interact with it in several different ways. These interactions depend on the nature of the light (its wavelength, frequency, energy, etc.) and the nature of the material. Light waves interact with an object by some combination of reflection, and transmittance with refraction. As such, an optically transparent material allows much of the light that falls on it to be transmitted, with little light being reflected. Materials which do not allow the transmission of light are called optically opaque or simply opaque.

In an embodiment, a silk fibroin material disclosed herein is optically transparent. In aspects of this embodiment, a silk fibroin material transmits, *e.g.,* about 75% of the light, about 80% of the light, about 85% of the light, about 90% of the light, about 95% of the light, or about 100% of the light. In other aspects of this embodiment, a silk fibroin material transmits, *e.g.,* at least 75% of the light, at least 80% of the light, at least 85% of the light, at least 90% of the light, or at least 95% of the light. In yet other aspects of this embodiment, a silk fibroin material transmits, *e.g.,* about 75% to about 100% of the light, about 80% to about 100% of the light, about 85% to about 100% of the light, about 90% to about 100% of the light, or about 95% to about 100% of the light.

In another embodiment, a silk fibroin material disclosed herein is optically opaque. In aspects of this embodiment, a silk fibroin material transmits, *e.g.,* about 5% of the light, about 10% of the light, about 15% of the light, about 20% of the light, about 25% of the light, about 30% of the light, about 35% of the light, about 40% of the light, about 45% of the light, about 50% of the light, about 55% of the light, about 60% of the light, about 65% of the light, or about 70% of the light. In other aspects of this embodiment, a silk fibroin material transmits, e.g., at most 5% of the light, at most 10% of the light, at most 15% of the light, at most 20% of the light, at most 25% of the light, at most 30% of the light, at most 35% of the light, at most 40% of the light, at most 45% of the light, at most 50% of the light, at most 55% of the light, at most 60% of the light, at most 65% of the light, at most 70% of the light, or at most 75% of the light. In other aspects of this embodiment, a silk fibroin material transmits, *e.g.,* about 5% to about 15%, about 5% to about 20%, about 5% to about 25%, about 5% to about 30%, about 5% to about 35%, about 5% to about 40%, about 5% to about 45%, about 5% to about 50%, about 5% to about 55%, about 5% to about 60%, about 5% to about 65%, about 5% to about 70%, about 5% to about 75%, about 15% to about 20%, about 15% to about 25%, about 15% to about 30%, about 15% to about 35%, about 15% to about 40%, about 15% to about 45%, about 15% to about 50%, about 15% to about 55%, about 15% to about 60%, about 15% to about 65%, about 15% to about 70%, about 15% to about 75%, about 25% to about 35%, about 25% to about 40%, about 25% to about 45%, about 25% to about 50%, about 25% to about 55%, about 25% to about 60%, about 25% to about 65%, about 25% to about 70%, or about 25% to about 75%, of the light.

In an embodiment, a silk fibroin material disclosed herein is optically translucent. In aspects of this embodiment, a silk fibroin material diffusely transmits, *e.g.,* about 75% of the light, about 80% of the light, about 85% of the light, about 90% of the light, about 95% of the light, or about 100% of the light. In other aspects of this embodiment, a silk fibroin material diffusely transmits, *e.g.,* at least 75% of the light, at least 80% of the light, at least 85% of the light, at least 90% of the light, or at least 95% of the light. In yet other aspects of this embodiment, a silk fibroin material diffusely transmits, *e.g.,* about 75% to about 100% of the light, about 80% to about 100% of the light, about 85% to about 100% of the light, about 90% to about 100% of the light, or about 95% to about 100% of the light.

A silk fibroin material disclosed herein may be further processed by pulverizing the silk fibroin material into particles and optionally mixed with a carrier phase such as, *e.g.,* water or a saline solution to form an injectable or topical substance like a solution, oil, lotion, gel, ointment, cream, slurry, salve, or paste. As such, the disclosed silk fibroin materials may be monophasic or multiphasic compositions. A silk fibroin material may be milled to a particle size from about 10 µm to about 1000 µm in diameter. In aspects of this embodiment, a particle size may be about 15 µm to about 30 µm, about 25 µm to about 50 µm, about 45 µm to about 75 µm, about 70 µm to about 100 µm, about 50 µm to about 200 µm, about 150 µm to about 300 µm, about 250 µm to about 400 µm, about 350 µm to about 500 µm, about 450 µm to about 600 µm, about 550 µm to about 700 µm, about 650 µm to about 800 µm, about 750 µm to about 900 µm, or about 850 µm to about 1000 µm. Saline is then added as a carrier phase by first determining the bulk volume of a silk fibroin material, then vigorously pulverizing the silk fibroin material into particles while incorporating an appropriate volume of saline to achieve a desired carrier to particle ratio. For example, silk fibroin material milling may be accomplished by means of a forced sieving of bulk silk fibroin material through a series of stainless steel cloth sieves of decreasing pore sizes.

Aspects of the present specification provide, in part, a silk fibroin material disclosed herein that exhibits a dynamic viscosity. Viscosity is resistance of a fluid to shear or flow caused by either shear stress or tensile stress. Viscosity describes a fluid's internal resistance to flow caused by intermolecular friction exerted when layers of fluids attempt to slide by one another and may be thought of as a measure of fluid friction. The less viscous the fluid, the greater its ease of movement (fluidity).

Viscosity can be defined in two ways; dynamic viscosity (µ, although η is sometimes used) or kinematic viscosity (v). Dynamic viscosity, also known as absolute or complex viscosity, is the tangential force per unit area required to move one horizontal plane with respect to the other at unit velocity when maintained a unit distance apart by the fluid. The SI physical unit of dynamic viscosity is the Pascal-second (Pa·s), which is identical to N·M⁻²·s. Dynamic viscosity can be expressed as T = µ dvx/dz, where T = shearing stress, µ = dynamic viscosity, and dvx/dz is the velocity gradient over time. For example, if a fluid with a viscosity of one Pa·s is placed between two plates, and one plate is pushed sideways with a shear stress of one Pascal, it moves a distance equal to the thickness of the layer between the plates in one second. Dynamic viscosity symbolize by is also used, is measured with various types of rheometers, devices used to measure the way in which a liquid, suspension or slurry flows in response to applied forces.

Kinematic viscosity (v) is the ratio of dynamic viscosity to density, a quantity in which no force is involved and is defined as follows: v = µ/p, where µ is the dynamic viscosity ρ is density with the SI unit of kg/m³. Kinematic viscosity is usually measured by a glass capillary viscometer as has an SI unit of m²/s.

The viscosity of a material is highly temperature dependent and for either dynamic or kinematic viscosity to be meaningful, the reference temperature must be quoted. For the viscosity values disclosed herein, a dynamic viscosity is measured at 1 Pa with a cone/plane geometry 2°/40 cm and a temperature of 20 °C. Examples of the dynamic viscosity of various fluids at 20 °C is as follows: water is about 1.0 x 10⁻³ Pa·s, blood is about 3-4 x 10⁻³ Pa·s, vegetable oil is about 60-85 x 10⁻³ Pa·s, motor oil SE 30 is about 0.2 Pa·s, glycerin is about 1.4 Pa·s, maple syrup is about 2-3 Pa·s, honey is about 10 Pa·s, chocolate syrup is about 10-25 Pa·s, peanut butter is about 150-250 Pa·s, lard is about 1,000 Pa·s, vegetable shortening is about 1,200 Pa.s, and tar is about 30,000 Pa·s.

In aspects of this embodiment, a silk fibroin material disclosed herein exhibits a dynamic viscosity of, *e.g.,* about 10 Pa·s, about 20 Pa·s, about 30 Pa·s, about 40 Pa·s, about 50 Pa·s, about 60 Pa·s, about 70 Pa·s, about 80 Pa·s, about 90 Pa·s, about 100 Pa·s, about 125 Pa·s, about 150 Pa·s, about 175 Pa.s, about 200 Pa·s, about 225 Pa·s, about 250 Pa·s, about 275 Pa·s, about 300 Pa·s, about 400 Pa·s, about 500 Pa·s, about 600 Pa·s, about 700 Pa·s, about 750 Pa·s, about 800 Pa·s, about 900 Pa·s, about 1,000 Pa·s, about 1,100 Pa·s, or about 1,200 Pa·s. In other aspects of this embodiment, a silk fibroin material disclosed herein exhibits a dynamic viscosity of, e.g., at most 10 Pa·s, at most 20 Pa.s, at most 30 Pa.s, at most 40 Pa·s, at most 50 Pa·s, at most 60 Pa·s, at most 70 Pa·s, at most 80 Pa·s, at most 90 Pa·s, at most 100 Pa·s, at most 125 Pa·s, at most 150 Pa·s, at most 175 Pa·s, at most 200 Pa·s, at most 225 Pa·s, at most 250 Pa·s, at most 275 Pa·s, at most 300 Pa·s, at most 400 Pa·s, at most 500 Pa·s, at most 600 Pa·s, at most 700 Pa·s, at most 750 Pa·s, at most 800 Pa·s, at most 900 Pa·s, or at most 1000 Pa·s. In yet other aspects of this embodiment, a silk fibroin material disclosed herein exhibits a dynamic viscosity of, e.g., about 10 Pa·s to about 100 Pa.s, about 10 Pa·s to about 150 Pa·s, about 10 Pa·s to about 250 Pa·s, about 50 Pa·s to about 100 Pa·s, about 50 Pa·s to about 150 Pa·s, about 50 Pa·s to about 250 Pa·s, about 100 Pa·s to about 500 Pa.s, about 100 Pa·s to about 750 Pa·s, about 100 Pa·s to about 1,000 Pa·s, about 100 Pa·s to about 1,200 Pa·s, about 300 Pa·s to about 500 Pa·s, about 300 Pa·s to about 750 Pa·s, about 300 Pa·s to about 1,000 Pa·s, or about 300 Pa·s to about 1,200 Pa·s.

Aspects of the present specification provide, in part, a silk fibroin material disclosed herein that exhibits cohesivity. Cohesivity, also referred to as cohesion cohesive attraction, cohesive force, or compression force is a physical property of a material, caused by the intermolecular attraction between like-molecules within the material that acts to unite the molecules. Cohesivity is expressed in terms of grams-force (gmf). Cohesiveness is affected by, among other factors, the molecular weight ratio of the initial free silk fibroin material polymer, the degree of crosslinking of silk fibroin material polymers, the amount of residual free silk fibroin material polymers following crosslinking, and the pH of the silk fibroin material. A composition should be sufficiently cohesive as to remain localized to a site of administration. Additionally, in certain applications, a sufficient cohesiveness is important for a composition to retain its shape, and thus functionality, in the event of mechanical load cycling. As such, in one embodiment, a silk fibroin material disclosed herein exhibits cohesivity, on par with water. In yet another embodiment, a silk fibroin material disclosed herein exhibits sufficient cohesivity to remain localized to a site of administration. In still another embodiment, a silk fibroin material disclosed herein exhibits sufficient cohesivity to retain its shape. In a further embodiment, a silk fibroin material disclosed herein exhibits sufficient cohesivity to retain its shape and functionality.

In aspects of this embodiment, a silk fibroin material disclosed herein has a cohesivity of, *e.g.,* about 10 gmf, about 20 gmf, about 30 gmf, about 40 gmf, about 50 gmf, about 60 gmf, about 70 gmf, about 80 gmf, about 90 gmf, about 100 gmf, about 150 gmf, or about 200 gmf. In other aspects of this embodiment, a silk fibroin material disclosed herein has a cohesivity of, *e.g.,* at least 10 gmf, at least 20 gmf, at least 30 gmf, at least 40 gmf, at least 50 gmf, at least 60 gmf, at least 70 gmf, at least 80 gmf, at least 90 gmf, at least 100 gmf, at least 150 gmf, or at least 200 gmf. In yet other aspects of this embodiment, a silk fibroin material disclosed herein has a cohesivity of, *e.g.,* at most 10 gmf, at most 20 gmf, at most 30 gmf, at most 40 gmf, at most 50 gmf, at most 60 gmf, at most 70 gmf, at most 80 gmf, at most 90 gmf, at most 100 gmf, at most 150 gmf, or at most 200 gmf. In yet other aspects of this embodiment, a silk fibroin material disclosed herein has a cohesivity of, *e.g.,* about 50 gmf to about 150 gmf, about 60 gmf to about 140 gmf, about 70 gmf to about 130 gmf, about 80 gmf to about 120 gmf, or about 90 gmf to about 110 gmf.

In yet other aspects of this embodiment, a silk fibroin material disclosed herein has a cohesivity of, *e.g.,* about 10 gmf to about 50 gmf, about 25 gmf to about 75 gmf, about 50 gmf to about 150 gmf, about 100 gmf to about 200 gmf, about 100 gmf to about 300 gmf, about 100 gmf to about 400 gmf, about 100 gmf to about 500 gmf, about 200 gmf to about 300 gmf, about 200 gmf to about 400 gmf, about 200 gmf to about 500 gmf, about 200 gmf to about 600 gmf, about 200 gmf to about 700 gmf, about 300 gmf to about 400 gmf, about 300 gmf to about 500 gmf, about 300 gmf to about 600 gmf, about 300 gmf to about 700 gmf, about 300 gmf to about 800 gmf, about 400 gmf to about 500, about 400 gmf to about 600, about 400 gmf to about 700, about 400 gmf to about 800, about 500 gmf to about 600 gmf, about 500 gmf to about 700 gmf, about 500 gmf to about 800 gmf, about 600 gmf to about 700 gmf, about 600 gmf to about 800 gmf, about 700 gmf to about 800 gmf, about 1000 gmf to about 2000 gmf, about 1000 gmf to about 3000 gmf, or about 2000 gmf to about 3000 gmf.

Aspects of the present specification provide, in part, a silk fibroin material disclosed herein that exhibits a physiologically-acceptable osmolarity. As used herein, the term "osmolarity" refers to the concentration of osmotically active solutes in solution. As used herein, the term "a physiologically-acceptable osmolarity" refers to an osmolarity in accord with, or characteristic of, the normal functioning of a living organism. As such, administration of a silk fibroin material as disclosed herein exhibits an osmolarity that has substantially no long term or permanent detrimental effect when administered to a mammal. Osmolarity is expressed in terms of osmoles of osmotically active solute per liter of solvent (Osmol/L or Osm/L). Osmolarity is distinct from molarity because it measures moles of osmotically active solute particles rather than moles of solute. The distinction arises because some compounds can dissociate in solution, whereas others cannot. The osmolarity of a solution can be calculated from the following expression: Osmol/L = Σ ϕᵢ ηᵢ Cᵢ, where ϕ is the osmotic coefficient, which accounts for the degree of non-ideality of the solution; η is the number of particles (e.g. ions) into which a molecule dissociates; and C is the molar concentration of the solute; and i is the index representing the identity of a particular solute. The osmolarity of a silk fibroin material disclosed herein can be measured using a conventional method that measures solutions.

In an embodiment, a silk fibroin material disclosed herein exhibits a physiologically-acceptable osmolarity. In aspects of this embodiment, a silk fibroin material exhibits an osmolarity of, *e.g.,* about 100 mOsm/L, about 150 mOsm/L, about 200 mOsm/L, about 250 mOsm/L, about 300 mOsm/L, about 350 mOsm/L, about 400 mOsm/L, about 450 mOsm/L, or about 500 mOsm/L. In other aspects of this embodiment, a silk fibroin material exhibits an osmolarity of, *e.g.,* at least 100 mOsm/L, at least 150 mOsm/L, at least 200 mOsm/L, at least 250 mOsm/L, at least 300 mOsm/L, at least 350 mOsm/L, at least 400 mOsm/L, at least 450 mOsm/L, or at least 500 mOsm/L. In yet other aspects of this embodiment, a silk fibroin material exhibits an osmolarity of, *e.g.,* at most 100 mOsm/L, at most 150 mOsm/L, at most 200 mOsm/L, at most 250 mOsm/L, at most 300 mOsm/L, at most 350 mOsm/L, at most 400 mOsm/L, at most 450 mOsm/L, or at most 500 mOsm/L. In still other aspects of this embodiment, a silk fibroin material exhibits an osmolarity of, *e.g.,* about 100 mOsm/L to about 500 mOsm/L, about 200 mOsm/L to about 500 mOsm/L, about 200 mOsm/L to about 400 mOsm/L, about 300 mOsm/L to about 400 mOsm/L, about 270 mOsm/L to about 390 mOsm/L, about 225 mOsm/L to about 350 mOsm/L, about 250 mOsm/L to about 325 mOsm/L, about 275 mOsm/L to about 300 mOsm/L, or about 285 mOsm/L to about 290 mOsm/L.

Aspects of the present specification provide, in part, a silk fibroin material disclosed herein that exhibits a physiologically-acceptable osmolality. As used herein, the term "osmolality" refers to the concentration of osmotically active solutes per kilo of solvent in the body. As used herein, the term "a physiologically-acceptable osmolality" refers to an osmolality in accord with, or characteristic of, the normal functioning of a living organism. As such, administration of a silk fibroin material disclosed herein exhibits an osmolality that has substantially no long term or permanent detrimental effect when administered to a mammal. Osmolality is expressed in terms of osmoles of osmotically active solute per kilogram of solvent (osmol/kg or Osm/kg) and is equal to the sum of the molalities of all the solutes present in that solution. The osmolality of a solution can be measured using an osmometer. The most commonly used instrument in modern laboratories is a freezing point depression osmometer. This instruments measure the change in freezing point that occurs in a solution with increasing osmolality (freezing point depression osmometer) or the change in vapor pressure that occurs in a solution with increasing osmolality (vapor pressure depression osmometer).

In an embodiment, a silk fibroin material disclosed herein exhibits a physiologically-acceptable osmolality. In aspects of this embodiment, a silk fibroin material exhibits an osmolality of, *e.g.,* about 100 mOsm/kg, about 150 mOsm/kg, about 200 mOsm/kg, about 250 mOsm/kg, about 300 mOsm/kg, about 350 mOsm/kg, about 400 mOsm/kg, about 450 mOsm/kg, or about 500 mOsm/kg. In other aspects of this embodiment, a silk fibroin material exhibits an osmolality of, *e.g.,* at least 100 mOsm/kg, at least 150 mOsm/kg, at least 200 mOsm/kg, at least 250 mOsm/kg, at least 300 mOsm/kg, at least 350 mOsm/kg, at least 400 mOsm/kg, at least 450 mOsm/kg, or at least 500 mOsm/kg. In yet other aspects of this embodiment, a silk fibroin material exhibits an osmolality of, *e.g.,* at most 100 mOsm/kg, at most 150 mOsm/kg, at most 200 mOsm/kg, at most 250 mOsm/kg, at most 300 mOsm/kg, at most 350 mOsm/kg, at most 400 mOsm/kg, at most 450 mOsm/kg, or at most 500 mOsm/kg. In still other aspects of this embodiment, a silk fibroin material exhibits an osmolality of, *e.g.,* about 100 mOsm/kg to about 500 mOsm/kg, about 200 mOsm/kg to about 500 mOsm/kg, about 200 mOsm/kg to about 400 mOsm/kg, about 300 mOsm/kg to about 400 mOsm/kg, about 270 mOsm/kg to about 390 mOsm/kg, about 225 mOsm/kg to about 350 mOsm/kg, about 250 mOsm/kg to about 325 mOsm/kg, about 275 mOsm/kg to about 300 mOsm/kg, or about 285 mOsm/kg to about 290 mOsm/kg.

A silk fibroin material disclosed herein is typically resistant to biodegradation upon administration to an individual. As used herein, the term "resistant to biodegradation" is synonymous with "resistant to bioerosion", "resistant to bioresorption", "non-biodegradable", "non-bioerodable" and "non-bioresorbable" and refers to a silk fibroin material disclosed herein that is not prone to degrading, eroding, resorbing, decomposing, or breaking down to any substantial or significant degree while implanted in an individual. This resistance to biodegradation enables the silk fibroin to retain and maintain the tissue space in order to promote new tissue growth into the space. Non-limiting examples of substantial non-degradation or resistance to biodegradation include less than 10% degradation of a silk fibroin material over a time period measured, less than 5% degradation of a silk fibroin material over a time period measured, less than 3% degradation of a silk fibroin material over a time period measured, less than 1% degradation of a silk fibroin material over a time period measured. In an embodiment, a silk fibroin material disclosed herein is substantially non-biodegradable or resistant to biodegradation upon administration to an individual.

However, it is anticipated that as the space becomes filled with regenerated tissue which will replace the space being occupied by the silk fibroin material. As such, the silk fibroin material should be degradable or time. The rate of degradation imparted on the silk fibroin material will be determined by the speed of replacement with fat and maintenance of volume. In one example, silk fibroin material will be resistant to degradation early on with degradation occurring over several months.

In aspects of this embodiment, a silk fibroin material is substantially non-biodegradable or resistance to biodegradation for, *e.g.,* about 10 days, about 20 days, about 30 days, about 40 days, about 50 days, about 60 days, about 70 days, about 80 days, or about 90 days, before biodegradation occurs. In other aspects of this embodiment, a silk fibroin material is substantially non-biodegradable or resistant to biodegradation for, *e.g.,* at least 10 days, at least 20 days, at least 30 days, at least 40 days, at least 50 days, at least 60 days, at least 70 days, at least 80 days, or at least 90 days, before biodegradation occurs. In yet other aspects of this embodiment, a silk fibroin material is substantially non-biodegradable or resistant to biodegradation for, *e.g.,* at most 10 days, at most 20 days, at most 30 days, at most 40 days, at most 50 days, at most 60 days, at most 70 days, at most 80 days, or at most 90 days, before biodegradation occurs. In still other aspects of this embodiment, a silk fibroin material is substantially non-biodegradable or resistant to biodegradation for, *e.g.,* about 10 days to about 30 days, about 20 days to about 50 days, about 40 days to about 60 days, about 50 days to about 80 days, or about 60 days to about 90 days, before biodegradation occurs.

In aspects of this embodiment, biodegradation of a silk fibroin material disclosed herein occurs with substantially first order release kinetics over a period of, e.g., about 7 days after, about 15 days, about 30 days, about 45 days, about 60 days, about 75 days, or about 90 days after degradation begins. In other aspects of this embodiment, biodegradation of a silk fibroin material disclosed herein occurs with substantially first order release kinetics over a period of *e.g.,* at least 7 days, at least 15 days, at least 30 days, at least 45 days, at least 60 days, at least 75 days, or at least 90 days after degradation begins. In yet other aspects of this embodiment, biodegradation of a silk fibroin material disclosed herein occurs with substantially first order release kinetics over a period of, *e.g.,* about 10 days to about 30 days, about 20 days to about 50 days, about 40 days to about 60 days, about 50 days to about 80 days, or about 60 days to about 90 days, after degradation begins.

The silk fibroin material described herein can be further modified after fabrication. For example, a silk scaffold can be coated with additive(s), such as bioactive substances that function as receptors or chemoattractors for a desired population of cells. The coating can be applied through absorption or chemical bonding. See, e.g. Kaplin, et al., Silk Biomaterials and Methods of Use Thereof, U.S. Patent 7,674,882; Kaplin, et al., Concentrated Aqueous Silk Fibroin Solution and Use Thereof, U.S. Patent 7,635,755; Kaplin, et al., Silk Fibroin Materials and Use Thereof, U.S. Patent Publication 2006/0273279; and Kaplin, et al., Silk Biomaterials and Methods of Use Thereof, International Patent Publication WO 2004/001103.

The addition of compound (c) to an adipose tissue, for instance, may be accomplished by any method that ensures sufficient distribution of the compound throughout the adipose tissue so as to promote formation of a blood supply sufficient to support the transplanted tissue. For example, mixing may occur through automated means, such as, *e.g.,* device-controlled agitation or centrifugation, or through manual methods, such as, *e.g.,* luer-locked syringes or vortexing.

The compositions disclosed herein may optionally comprise one or more additional materials useful as a filler for tissue space. Exemplary filler materials suitable with the compositions and methods disclosed herein include, without limitation, fillers comprising a polypeptide such as, e.g., a resilin, a resilin-like polypeptide, an elastin, an elastin-like polypeptide, a silk protein-elastin-like polypeptide, an abductin, a byssus, a gliadin, a glutenin, abductin, keratin, gelatin, or collagen; fillers comprising a polysaccharide such as, e.g., cellulose, agarose, chitosan, chitin, or a glycosaminoglycan like chondroitin sulfate, dermatan sulfate, keratan sulfate, or hyaluronic acid; and fillers comprising a polyester such as, e.g., D-lactic acid, L-lactic acid, racemic lactic acid, glycolic acid, caprolactone. These additional tissue space fillers may be administered as a separate component, or may be complexed with a silk fibroin material disclosed herein.

The compositions disclosed herein may optionally comprise one or more immunosuppressive agents that reduce, and preferably prevent, rejection of the transplanted tissue. As used herein, the term "immunosuppressive agent" is synonymous with "immunosuppressive drug" and refers to a compound that inhibits or interferes with normal immune function. Exemplary immunosuppressive agents suitable with the compositions and methods disclosed herein include, without limitation, agents that inhibit T-cell/B-cell costimulation pathways like agents that interfere with the coupling of T-cells and B-cells via the CTLA4 and B7 pathways, including cyclosporine A, myophenylate mofetil, rapamicin, and anti-thymocyte globulin. An immunosuppressive drug is administered in a formulation that is compatible with the route of administration and is administered to an individual at a dosage sufficient to achieve the desired therapeutic effect.

In aspects of this embodiment, a composition disclosed herein comprises an immunosuppressive agent in an amount of, *e.g.,* about 0.1 %, about 0.2%, about 0.3%, about 0.4%, about 0.5%, about 0.6%, about 0.7%, about 0.8% about 0.9%, about 1.0%, about 2.0%, about 3.0%, about 4.0%, about 5.0%, about 6.0%, about 7.0%, about 8.0%, about 9.0%, or about 10% by weight of the total composition. In other aspects, a composition disclosed herein comprises an immunosuppressive agent in an amount of, e.g., at least 0.1%, at least 0.2%, at least 0.3%, at least 0.4%, at least 0.5%, at least 0.6%, at least 0.7%, at least 0.8% at least 0.9%, at least 1.0%, at least 2.0%, at least 3.0%, at least 4.0%, at least 5.0%, at least 6.0%, at least 7.0%, at least 8.0%, at least 9.0%, or at least 10% by weight of the total composition. In yet other aspects, a composition disclosed herein comprises an immunosuppressive agent in an amount of, *e.g.,* at most 0.1%, at most 0.2%, at most 0.3%, at most 0.4%, at most 0.5%, at most 0.6%, at most 0.7%, at most 0.8% at most 0.9%, at most 1.0%, at most 2.0%, at most 3.0%, at most 4.0%, at most 5.0%, at most 6.0%, at most 7.0%, at most 8.0%, at most 9.0%, or at most 10% by weight of the total composition. In further aspects, a composition disclosed herein comprises an anti-inflammatory agent in an amount of, e.g., about 0.1% to about 0.5%, about 0.1% to about 1.0%, about 0.1% to about 2.0%, about 0.1 % to about 3.0%, about 0.1 % to about 4.0%, about 0.1 % to about 5.0%, about 0.2% to about 0.9%, about 0.2% to about 1.0%, about 0.2% to about 2.0%, about 0.5% to about 1.0%, or about 0.5% to about 2.0% by weight of the total composition.

In other aspects of this embodiment, a composition disclosed herein comprises an immunosuppressive agent at a concentration of, *e.g.,* about 0.01 mg/mL, about 0.02 mg/mL, about 0.03 mg/mL, about 0.04 mg/mL, about 0.05 mg/mL, about 0.06 mg/mL, about 0.07 mg/mL, about 0.08 mg/mL, about 0.09 mg/mL, about 0.1 mg/mL, about 0.2 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL, about 1.0 mg/mL, about 2.0 mg/mL, about 3.0 mg/mL, about 4.0 mg/mL, about 5.0 mg/mL, about 6.0 mg/mL, about 7.0 mg/mL, about 8.0 mg/mL, about 9.0 mg/mL, or about 10 mg/mL. In yet other aspects of this embodiment, a composition disclosed herein comprises an immunosuppressive agent at a concentration of, *e.g.,* at least 0.01 mg/mL, at least 0.02 mg/mL, at least 0.03 mg/mL, at least 0.04 mg/mL, at least 0.05 mg/mL, at least 0.06 mg/mL, at least 0.07 mg/mL, at least 0.08 mg/mL, at least 0.09 mg/mL, at least 0.1 mg/mL, at least 0.2 mg/mL, at least 0.3 mg/mL, at least 0.4 mg/mL, at least 0.5 mg/mL, at least 0.6 mg/mL, at least 0.7 mg/mL, at least 0.8 mg/mL, at least 0.9 mg/mL, at least 1.0 mg/mL, at least 2.0 mg/mL, at least 3.0 mg/mL, at least 4.0 mg/mL, at least 5.0 mg/mL, at least 6.0 mg/mL, at least 7.0 mg/mL, at least 8.0 mg/mL, at least 9.0 mg/mL, or at least 10 mg/mL. In still other aspects of this embodiment, a composition disclosed herein comprises an immunosuppressive agent at a concentration of, *e.g.,* at most 0.01 mg/mL, at most 0.02 mg/mL, at most 0.03 mg/mL, at most 0.04 mg/mL, at most 0.05 mg/mL, at most 0.06 mg/mL, at most 0.07 mg/mL, at most 0.08 mg/mL, at most 0.09 mg/mL, at most 0.1 mg/mL, at most 0.2 mg/mL, at most 0.3 mg/mL, at most 0.4 mg/mL, at most 0.5 mg/mL, at most 0.6 mg/mL, at most 0.7 mg/mL, at most 0.8 mg/mL, at most 0.9 mg/mL, at most 1.0 mg/mL, at most 2.0 mg/mL, at most 3.0 mg/mL, at most 4.0 mg/mL, at most 5.0 mg/mL, at most 6.0 mg/mL, at most 7.0 mg/mL, at most 8.0 mg/mL, at most 9.0 mg/mL, or at most 10 mg/mL. In further aspects, a composition disclosed herein comprises an anti-inflammatory agent at a concentration of, *e.g.,* about 0.01 mg/mL to about 0.7 mg/mL, about 0.06 mg/mL to about 0.7 mg/mL, about 0.01 mg/mL to about 1.0 mg/mL, about 0.05 mg/mL to about 1.0 mg/mL, about 0.06 mg/mL to about 1.0 mg/mL, about 0.1 mg/mL to about 1.0 mg/mL, about 0.1 mg/mL to about 2.0 mg/mL, about 0.1 mg/mL to about 3.0 mg/mL, about 0.1 mg/mL to about 4.0 mg/mL, about 0.1 mg/mL to about 5.0 mg/mL, about 0.2 mg/mL to about 0.9 mg/mL, about 0.2 mg/mL to about 1.0 mg/mL, about 0.2 mg/mL to about 2.0 mg/mL, about 0.5 mg/mL to about 1.0 mg/mL, or about 0.5 mg/mL to about 2.0 mg/mL.

In another embodiment, a composition disclosed herein does not comprise an immunosuppressive agent.

A compound disclosed herein, or a composition comprising such a compound is generally administered to an individual as a pharmaceutical composition. Pharmaceutical compositions may be prepared by combining a therapeutically effective amount of at least one compound according to the present specification, or a pharmaceutically acceptable acid addition salt thereof, as an active ingredient, with conventional acceptable pharmaceutical excipients, and by preparation of unit dosage forms. The therapeutically efficient amount typically is between about 0.0001% (w/v) and about 5% (w/v), preferably about 0.001% (w/v) to about 1.0% (w/v) in liquid formulations. As used herein, the term "pharmaceutical composition" refers to a therapeutically effective concentration of an active compound, such as, *e.g.,* any of the compounds disclosed herein. Preferably, the pharmaceutical composition does not produce an adverse, allergic, or other untoward or unwanted reaction when administered to an individual. A pharmaceutical composition disclosed herein is useful for medical and veterinary applications. A pharmaceutical composition may be administered to an individual alone, or in combination with other supplementary active compounds, agents, drugs or hormones. The pharmaceutical compositions may be manufactured using any of a variety of processes, including, without limitation, conventional mixing, dissolving, granulating, dragee-making, levigating, emulsifying, encapsulating, entrapping, and lyophilizing. The pharmaceutical composition can take any of a variety of forms including, without limitation, a sterile solution, suspension, emulsion, lyophilizate, tablet, pill, pellet, capsule, powder, syrup, elixir, or any other dosage form suitable for administration.

The compounds disclosed herein may also be incorporated into a drug delivery platform in order to achieve a controlled compound release profile over time. Such a drug delivery platform comprises a compound disclosed herein dispersed within a polymer matrix, typically a biodegradable, bioerodible, and/or bioresorbable polymer matrix. As used herein, the term "polymer" refers to synthetic homo- or copolymers, naturally occurring homo- or copolymers, as well as synthetic modifications or derivatives thereof having a linear, branched or star structure. Copolymers can be arranged in any form, such as, *e.g.,* random, block, segmented, tapered blocks, graft, or triblock. Polymers are generally condensation polymers. Polymers can be further modified to enhance their mechanical or degradation properties by introducing cross-linking agents or changing the hydrophobicity of the side residues. If crosslinked, polymers are usually less than 5% crosslinked, usually less than 1% crosslinked.

Suitable polymers for use in a drug delivery platform include, without limitation, alginates, aliphatic polyesters, polyalkylene oxalates, polyamides, polyamidoesters, polyanhydrides, polycarbonates, polyesters, polyethylene glycol, polyhydroxyaliphatic carboxylic acids, polyorthoesters, polyoxaesters, polypeptides, polyphosphazenes, polysaccharides, and polyurethanes. The polymer usually comprises at least about 10% (w/w), at least about 20% (w/w), at least about 30% (w/w), at least about 40% (w/w), at least about 50% (w/w), at least about 60% (w/w), at least about 70% (w/w), at least about 80% (w/w), or at least about 90% (w/w) of the drug delivery platform. Examples of biodegradable, bioerodible, and/or bioresorbable polymers and methods useful to make a drug delivery platform are described in, *e.g.,* Drost, et. al., Controlled Release Formulation, U.S. Patent 4,756,911; Smith, et. al., Sustained Release Drug Delivery Devices, U.S. Patent 5,378,475; Wong and Kochinke, Formulation for Controlled Release of Drugs by Combining Hyrophilic and Hydrophobic Agents, U.S. Patent 7,048,946; Hughes, et. Al., Compositions and Methods for Localized Therapy of the Eye, U.S. Patent Publication 2005/0181017; Hughes, Hypotensive Lipid-Containing Biodegradable Intraocular Implants and Related Methods, U.S. Patent Publication 2005/0244464; and Altman, et al., Silk Fibroin Hydrogels and Uses Thereof, U.S. Patent Publication 2011/0008437.

In aspects of this embodiment, a polymer composing the matrix is a polypeptide such as, *e.g.,* silk fibroin (discussed *supra*), keratin, or collagen. In other aspects of this embodiment, a polymer composing the matrix is a polysaccharide such as, *e.g.,* cellulose, agarose, elastin, chitosan, chitin, or a glycosaminoglycan like chondroitin sulfate, dermatan sulfate, keratan sulfate, or hyaluronic acid. In yet other aspects of this embodiment, a polymer composing the matrix is a polyester such as, *e.g.,* D-lactic acid, L-lactic acid, racemic lactic acid, glycolic acid, caprolactone, and combinations thereof.

One of ordinary skill in the art appreciates that the selection of a suitable polymer for forming a suitable disclosed drug delivery platform depends on several factors. The more relevant factors in the selection of the appropriate polymer(s), include, without limitation, compatibility of polymer with drug, desired release kinetics of drug, desired biodegradation kinetics of platform at implantation site, desired bioerodible kinetics of platform at implantation site, desired bioresorbable kinetics of platform at implantation site, *in vivo* mechanical performance of platform, processing temperatures, biocompatibility of platform, and patient tolerance. Other relevant factors that, to some extent, dictate the *in vitro* and *in vivo* behavior of the polymer include the chemical composition, spatial distribution of the constituents, the molecular weight of the polymer and the degree of crystallinity.

A drug delivery platform includes both a sustained release drug delivery platform and an extended release drug delivery platform. As used herein, the term "sustained release" refers to the release of a compound disclosed herein over a period of about seven days or more. As used herein, the term "extended release" refers to the release of a compound disclosed herein over a period of time of less than about seven days.

In aspects of this embodiment, a sustained release drug delivery platform releases a compound disclosed herein with substantially first order release kinetics over a period of, *e.g.,* about 7 days after administration, about 15 days after administration, about 30 days after administration, about 45 days after administration, about 60 days after administration, about 75 days after administration, or about 90 days after administration. In other aspects of this embodiment, a sustained release drug delivery platform releases a compound disclosed herein with substantially first order release kinetics over a period of, *e.g.,* at least 7 days after administration, at least 15 days after administration, at least 30 days after administration, at least 45 days after administration, at least 60 days after administration, at least 75 days after administration, or at least 90 days after administration.

In aspects of this embodiment, a drug delivery platform releases a compound disclosed herein with substantially first order release kinetics over a period of, e.g., about 1 day after administration, about 2 days after administration, about 3 days after administration, about 4 days after administration, about 5 days after administration, or about 6 days after administration. In other aspects of this embodiment, a drug delivery platform releases a compound disclosed herein with substantially first order release kinetics over a period of, *e.g.,* at most 1 day after administration, at most 2 days after administration, at most 3 days after administration, at most 4 days after administration, at most 5 days after administration, or at most 6 days after administration.

A pharmaceutical composition disclosed herein can optionally include a pharmaceutically acceptable carrier that facilitates processing of an active compound into pharmaceutically acceptable compositions. As used herein, the term "pharmacologically acceptable carrier" is synonymous with "pharmacological carrier" and refers to any carrier that has substantially no long term or permanent detrimental effect when administered and encompasses terms such as "pharmacologically acceptable vehicle, stabilizer, diluent, additive, auxiliary, or excipient." Such a carrier generally is mixed with an active compound or permitted to dilute or enclose the active compound and can be a solid, semi-solid, or liquid agent. It is understood that the active compounds can be soluble or can be delivered as a suspension in the desired carrier or diluent. Any of a variety of pharmaceutically acceptable carriers can be used including, without limitation, aqueous media such as, *e.g.,* water, saline, glycine, hyaluronic acid and the like; solid carriers such as, *e.g.,* starch, magnesium stearate, mannitol, sodium saccharin, talcum, cellulose, glucose, sucrose, lactose, trehalose, magnesium carbonate, and the like; solvents; dispersion media; coatings; antibacterial and antifungal agents; isotonic and absorption delaying agents; or any other inactive ingredient. Selection of a pharmacologically acceptable carrier can depend on the mode of administration. Except insofar as any pharmacologically acceptable carrier is incompatible with the active compound, its use in pharmaceutically acceptable compositions is contemplated. Non-limiting examples of specific uses of such pharmaceutical carriers can be found in Pharmaceutical Dosage Forms and Drug Delivery Systems (Howard C. Ansel et al., eds., Lippincott Williams & Wilkins Publishers, 7th ed. 1999); Remington: The Science and Practice of Pharmacy (Alfonso R. Gennaro ed., Lippincott, Williams & Wilkins, 20th ed. 2000); Goodman & Gilman's The Pharmacological Basis of Therapeutics (Joel G. Hardman et al., eds., McGraw-Hill Professional, 10th ed. 2001); and Handbook of Pharmaceutical Excipients (Raymond C. Rowe et al., APhA Publications, 4th edition 2003). These protocols are routine and any modifications are well within the scope of one skilled in the art and from the teaching herein.

A pharmaceutical composition disclosed herein may optionally include, without limitation, other pharmaceutically acceptable components (or pharmaceutical components), including, without limitation, buffers, preservatives, tonicity adjusters, salts, antioxidants, osmolality adjusting agents, physiological substances, pharmacological substances, bulking agents, emulsifying agents, wetting agents, sweetening or flavoring agents, and the like. Various buffers and means for adjusting pH can be used to prepare a pharmaceutical composition disclosed herein, provided that the resulting preparation is pharmaceutically acceptable. Such buffers include, without limitation, acetate buffers, borate buffers, citrate buffers, phosphate buffers, neutral buffered saline, and phosphate buffered saline. It is understood that acids or bases can be used to adjust the pH of a composition as needed. Pharmaceutically acceptable antioxidants include, without limitation, sodium metabisulfite, sodium thiosulfate, acetylcysteine, butylated hydroxyanisole, and butylated hydroxytoluene. Useful preservatives include, without limitation, benzalkonium chloride, chlorobutanol, thimerosal, phenylmercuric acetate, phenylmercuric nitrate, a stabilized oxy chloro composition, such as, *e.g.,* sodium chlorite and chelants, such as, *e.g.,* DTPA or DTPA-bisamide, calcium DTPA, and CaNaDTPA-bisamide. Tonicity adjustors useful in a pharmaceutical composition include, without limitation, salts such as, *e.g.,* sodium chloride, potassium chloride, mannitol or glycerin and other pharmaceutically acceptable tonicity adjustor. The pharmaceutical composition may be provided as a salt and can be formed with many acids, including but not limited to, hydrochloric, sulfuric, acetic, lactic, tartaric, malic, and succinic acid. Salts tend to be more soluble in aqueous or other protonic solvents than are the corresponding free base forms. It is understood that these and other substances known in the art of pharmacology can be included in a pharmaceutical composition useful in the invention.

As used herein, the term "treating," refers to reducing or eliminating in an individual a cosmetic or clinical symptom of a soft tissue condition characterized by a soft tissue imperfection, defect, disease, and/or disorder; or delaying or preventing in an individual the onset of a cosmetic or clinical symptom of a condition characterized by a soft tissue imperfection, defect, disease, and/or disorder. For example, the term "treating" can mean reducing a symptom of a condition characterized by a soft tissue defect, disease, and/or disorder by, *e.g.,* at least 20%, at least 30%, at least 40%, at least 50%, at least 60%, at least 70%, at least 80%, at least 90% or at least 100%. The effectiveness of a compound disclosed herein in treating a condition characterized by a soft tissue defect, disease, and/or disorder can be determined by observing one or more cosmetic, clinical symptoms, and/or physiological indicators associated with the condition. An improvement in a soft tissue defect, disease, and/or disorder also can be indicated by a reduced need for a concurrent therapy. Those of skill in the art will know the appropriate symptoms or indicators associated with specific soft tissue defect, disease, and/or disorder and will know how to determine if an individual is a candidate for treatment with a compound or composition disclosed herein.

A composition or compound is administered to an individual. An individual is typically a human being. Typically, any individual who is a candidate for a conventional soft tissue replacement procedure is a candidate for a soft tissue replacement procedure disclosed herein. In addition, the presently disclosed compositions and methods may apply to individuals seeking a small/moderate enlargement, shape change or contour alteration of a body part or region, which may not be technically possible or aesthetically acceptable with existing soft tissue implant technology. Preoperative evaluation typically includes routine history and physical examination in addition to thorough informed consent disclosing all relevant risks and benefits of the procedure.

The composition of the invention is useful in treating a soft tissue condition. A soft tissue condition includes, without limitation, a soft tissue imperfection, defect, disease, and/or disorder. Non-limiting examples of a soft tissue condition include a breast imperfection, defect, disease and/or disorder, such as, *e.g.,* a breast augmentation, a breast reconstruction mastopexy, micromastia, thoracic hypoplasia, Poland's syndrome, defects due to implant complications like capsular contraction and/or rupture; a facial imperfection, defect, disease or disorder, such as, *e.g.,* a facial augmentation, a facial reconstruction, Parry-Romberg syndrome, lupus erythematosus profundus, dermal divots, sunken cheeks, thin lips, nasal imperfections or defects, retro-orbital imperfections or defects, a facial fold, line and/or wrinkle like a glabellar line, a nasolabial line, a perioral line, and/or a marionette line, and/or other contour deformities or imperfections of the face; a neck imperfection, defect, disease or disorder; a skin imperfection, defect, disease and/or disorder; other soft tissue imperfections, defects, diseases and/or disorders, such as, *e.g.,* an augmentation or a reconstruction of the upper arm, lower arm, hand, shoulder, back, torso including abdomen, buttocks, upper leg, lower leg including calves, foot including plantar fat pad, eye, genitals, or other body part, region or area, or a disease or disorder affecting these body parts, regions or areas; urinary incontinence, fecal incontinence, other forms of incontinence; and gastroesophageal reflux disease (GERD).

The amount of adipose tissue and/or silk fibroin material used with any of the methods as disclosed herein will typically be determined based on the alteration and/or improvement desired, the reduction and/or elimination of a soft tissue condition symptom desired, the clinical and/or cosmetic effect desired by the individual and/or physician, and the body part or region being treated. The effectiveness of adipose tissue and/or silk fibroin material administration may be manifested by one or more of the following clinical and/or cosmetic measures: altered and/or improved soft tissue shape, altered and/or improved soft tissue size, altered and/or improved soft tissue contour, altered and/or improved tissue function, improved transplant tissue survival, improved patient satisfaction and/or quality of life, and decreased use of implantable foreign material.

For example, for breast augmentation procedures, effectiveness of adipose tissue and/or silk fibroin material administration may be manifested by one or more of the following clinical and/or cosmetic measures: increased breast size, altered breast shape, altered breast contour, sustained engraftment, decreased rate of liponecrotic cyst formation, improved patient satisfaction and/or quality of life, and decreased use of breast implant.

As another example, effectiveness of adipose tissue and/or silk fibroin material administration in treating a facial soft tissue may be manifested by one or more of the following clinical and/or cosmetic measures: increased size, shape, and/or contour of facial feature like increased size, shape, and/or contour of lip, cheek or eye region; altered size, shape, and/or contour of facial feature like altered size, shape, and/or contour of lip, cheek or eye region shape; reduction or elimination of a wrinkle, fold or line in the skin; resistance to a wrinkle, fold or line in the skin; rehydration of the skin; increased elasticity to the skin; reduction or elimination of skin roughness; increased and/or improved skin tautness; reduction or elimination of stretch lines or marks; increased and/or improved skin tone, shine, brightness and/or radiance; increased and/or improved skin color, reduction or elimination of skin paleness; sustained engraftment of composition; decreased side effects; improved patient satisfaction and/or quality of life.

As yet another example, for urinary incontinence procedures, effectiveness of adipose tissue and/or silk fibroin material administration for sphincter support may be manifested by one or more of the following clinical measures: decreased frequency of incontinence, sustained engraftment, improved patient satisfaction and/or quality of life, and decreased use of implantable foreign filler.

The amount of compound (c) will typically be a therapeutically effective amount. As used herein, the term "therapeutically effective amount" is synonymous with "effective amount", "therapeutically effective dose", and/or " effective dose" and refers to the amount of compound that will elicit the biological, cosmetic or clinical response being sought by the practitioner in an individual in need thereof. As a non-limiting example, an effective amount is an amount sufficient to promote formation of a blood supply sufficient to support the transplanted tissue. As another non-limiting example, an effective amount is an amount sufficient to promote formation of new blood vessels and associated vasculature (angiogenesis) and/or an amount sufficient to promote repair or remodeling of existing blood vessels and associated vasculature. The appropriate effective amount to be administered for a particular application of the disclosed methods can be determined by those skilled in the art, using the guidance provided herein. For example, an effective amount can be extrapolated from *in vitro* and *in vivo* assays as described in the present specification. One skilled in the art will recognize that the condition of the individual can be monitored throughout the course of therapy and that the effective amount of a compound or composition disclosed herein that is administered can be adjusted accordingly.

In aspects of this embodiment, the amount of a compound added is, e.g., 0.01 mg, 0.05 mg, 0.1 mg, 0.5 mg, 1 mg, 5 mg, 10 mg, 20 mg, 30 mg, 40 mg, 50 mg, 60 mg, 70 mg, 80 mg, 90 mg, 100 mg, 150 mg, 200 mg, 250 mg, 500 mg, 750 mg, or 1000 mg. In other aspects of this embodiment, the amount of a compound added is, *e.g.,* 0.01 mg/mL, 0.05 mg/mL, 0.1 mg/mL, 0.5 mg/mL, 1 mg/mL, 5 mg/mL, 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 150 mg/mL, 200 mg/mL, 250 mg/mL, 500 mg/mL, 750 mg/mL, or 1000 mg/mL. In yet other aspects of this embodiment, the amount of a compound added is, *e.g.,* 0.01 mg/10 mL of tissue, 0.05 mg/10 mL of tissue, 0.1 mg/10 mL of tissue, 0.5 mg/10 mL of tissue, 1 mg/10 mL of tissue, 5 mg/10 mL of tissue, 10 mg/10 mL of tissue, 20 mg/10 mL of tissue, 30 mg/10 mL of tissue, 40 mg/10 mL of tissue, 50 mg/10 mL of tissue, 60 mg/10 mL of tissue, 70 mg/10 mL of tissue, 80 mg/10 mL of tissue, 90 mg/10 mL of tissue, 100 mg/10 mL of tissue, 150 mg/10 mL of tissue, 200 mg/10 mL of tissue, 250 mg/10 mL of tissue, 500 mg/10 mL of tissue, 750 mg/10 mL of tissue, or 1000 mg/10 mL of tissue.

In aspects of this embodiment, the amount of a compound added is, *e.g.,* about 0.01 mg to about 0.1 mg, about 0.1 mg to about 1 mg, about 1 mg to about 10 mg, about 10 mg to about 100 mg, or about 100 mg to about 1000 mg. In other aspects of this embodiment, the amount of a compound added is, *e.g.,* about 0.01 mg/mL to about 0.1 mg/mL, about 0.1 mg/mL to about 1 mg/mL, about 1 mg/mL to about 10 mg/mL, about 10 mg/mL to about 100 mg/mL, or about 100 mg/mL to about 1000 mg/mL. In yet other aspects of this embodiment, the amount of a compound added is, e.g., about 0.01 mg/10 mL of tissue to about 0.1 mg/10 mL of tissue, about 0.1 mg/10 mL of tissue to about 1 mg/10 mL of tissue, about 1 mg/10 mL of tissue to about 10 mg/10 mL of tissue, about 10 mg/10 mL of tissue to about 100 mg/10 mL of tissue, or about 100 mg/10 mL of tissue to about 1000 mg/10 mL of tissue.

In aspects of this embodiment, the amount of a compound added is, *e.g.,* about 0.0001% (w/v) to about 5% (w/v), about 0.001% (w/v) to about 5% (w/v), about 0.01% (w/v) to about 5% (w/v), about 0.1% (w/v) to about 5% (w/v), or about 1% (w/v) to about 5% (w/v). In other aspects of this embodiment, the amount of a compound added is, *e.g*., about 0.001% (w/v) to about 1.0% (w/v), about 0.01% (w/v) to about 1.0% (w/v), or about 0.1 % (w/v) to about 1.0% (w/v).

A composition comprising compound (c) and/or silk fibroin can be mixed with harvested adipose tissue and, as such, be administered simultaneously with the adipose tissue. Additionally, or alternatively, a composition comprising a compound and/or silk fibroin, as disclosed herein, can be administered after administration of the adipose tissue. In this embodiment, the composition can be administered to the individual additionally, or alternatively, *e.g.,* about 1 day after adipose tissue implantation, about 2 days after adipose tissue implantation, about 3 days after adipose tissue implantation, or about 4 days after adipose tissue implantation. In other aspects, a composition disclosed herein is administered to the individual additionally, or alternatively, *e.g.,* about once a day for about 3 to about 10 consecutive days, about once a day for about 7 to about 14 consecutive days, about once a day for about 10 to about 21 consecutive days, or about once a day for about 14 to about 28 consecutive days. In other aspects, the daily dose can be, *e.g.,* twice a day, three times a day, or four times a day.

The route of administration of composition administered to an individual patient will typically be determined based on the cosmetic and/or clinical effect desired by the individual and/or physician and the body part or region being treated. Compositions or adipose tissue may be administered by any means known to persons of ordinary skill in the art including, without limitation, syringe with needle, catheter, or by direct surgical implantation. In addition, the disclosed composition or adipose tissue can be administered once, or over a plurality of times. Ultimately, the timing used will follow quality care standards.

For a breast soft tissue replacement procedure, the route of administration may include axillary, periareolar, and/or inframammary routes. For a facial soft tissue replacement procedure, the route of administration can be frontal, temporal, zygomatic, periocular, amdibula, perioral or chin routes. In urinary incontinence procedures, the route of administration may include transurethral or periurethral routes. Alternatively or in addition, the transplant may be delivered via an antegrade route. The routes discussed herein do not exclude the use of multiple routes to achieve the desired clinical effect, or umbilical incision. Alternatively or in addition, cell-enhanced tissue may be delivered through a transaxillary endoscopic subpectoral approach.

A composition or compound administered to an individual to treat a soft tissue condition promotes formation of a blood supply sufficient to support the transplanted tissue. Blood supply formation includes, without limitation, formation of new blood vessels and associated vasculature (angiogenesis) and repair or remodel of existing blood vessels and associated vasculature.

Treatment of a soft tissue condition can comprise the steps of a) administering adipose tissue to a site of the soft tissue condition; and b) administering a composition comprising a compound as disclosed herein to the site of the soft tissue condition. In certain aspects, a composition comprising silk fibroin is administered in step (a) or, alternately, in step (b). The adipose tissue, compound(s), and silk fibroin administered include the adipose tissue disclosed herein. In these methods, the adipose tissue is administered separately from a composition comprising the compound and/or silk fibroin. Thus, in one aspect of this embodiment, adipose tissue is administered first, followed by administration of a composition comprising the compound and silk fibroin. In another aspect of this embodiment, one or more compositions comprising a compound and silk fibroin (i.e., in the same composition or a separate composition) are administered first, followed by administration of adipose tissue. In certain aspects, compositions comprising one or more compounds further comprise silk fibroin. In other embodiments, a second composition comprising silk fibroin is administered at the same time, before, or after the administration of the first composition that comprises one or more of the compounds disclosed herein.

## Claims

1. A composition comprising:
(a) an adipose tissue;
(b) a silk fibroin material which comprises no more than 1% (w/w) of sericin; and
(c) a compound represented by the following formula IX or a pharmaceutically-acceptable salt thereof: 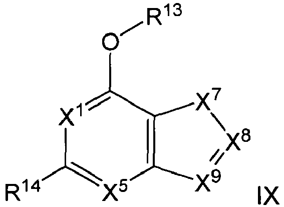 wherein:
X¹ and X⁵ are independently CH or N;
X⁷ is NH, O, or S;
X⁸ and X⁹ are independently CH or N;
R¹³ is optionally-substituted aryl or optionally-substituted heteroaryl; and
R¹⁴ is C₁₋₆ alkyl, optionally-substituted aryl, or optionally-substituted heteroaryl.

2. The composition of Claim 1, wherein compound (c) is a compound represented by the following formula X or a pharmaceutically-acceptable salt thereof: 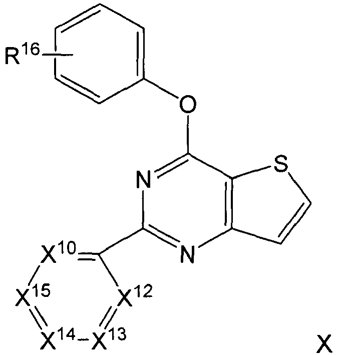 wherein:
X¹⁰, X¹², X¹³, X¹⁴, and X¹⁵ are each independently CR¹⁹ or N;
R¹⁶ is independently H, OH, O, F, Cl, Br, I, C₁₋₆ alkyl, -O-C₁₋₆ alkyl, CN, or C₁₋₆ alkyl-CN; and
R¹⁹ is independently H, OH, O, F, Cl, Br, I, C₁₋₁₀ alkyl, -O-C₁₋₁₀ alkyl, or -O-C₄₋₁₀ alkyl-heterocyclyl.

3. The composition of Claim 1, wherein compound (c) is a compound represented by the following formula XI or a pharmaceutically-acceptable salt thereof: 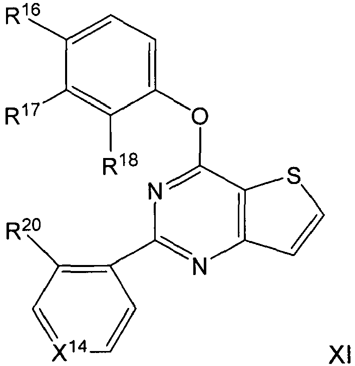 wherein:
X¹⁴ is CR¹⁹ or N;
R¹⁶ , R¹⁷ , and R¹⁸ are each independently H, OH, O, F, Cl, Br, I, C₁₋₆ alkyl, -O-C₁₋₆ alkyl, CN, or C₁₋₆ alkyl-CN;
R¹⁹ is H, OH, O, F, Cl, Br, I, C₁₋₁₀ alkyl, -O-C₁₋₁₀ alkyl, or 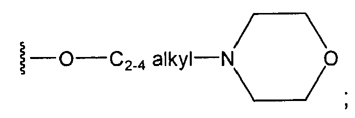 and
R²⁰ is H, F, Cl, Br, or I.

4. The composition of Claim 1, wherein compound (c) is one of the following compounds or a pharmaceutically-acceptable salt thereof:

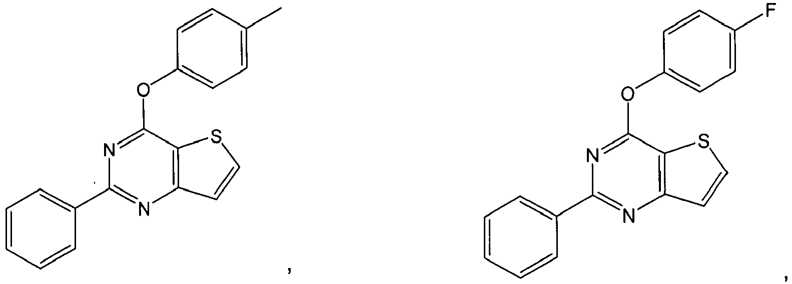

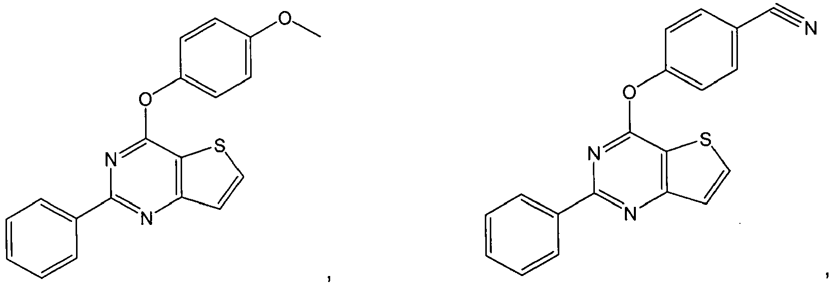

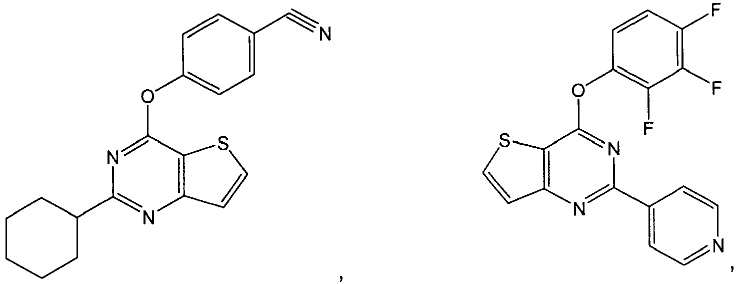

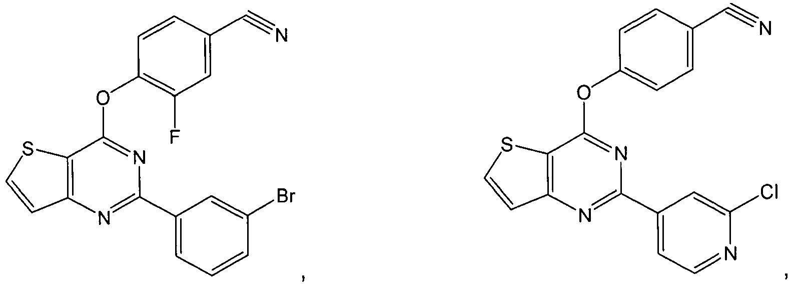

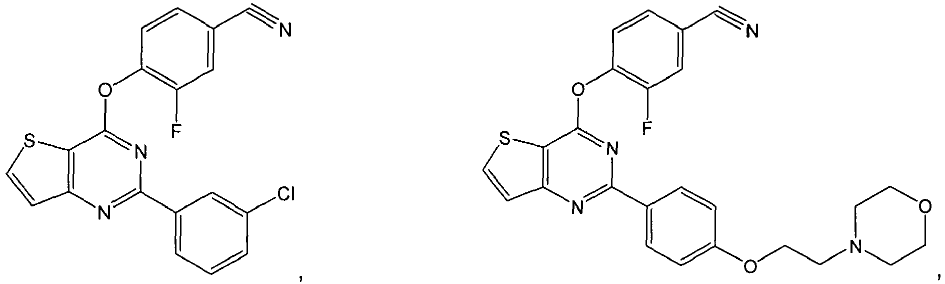

5. The composition of Claim 1, wherein compound (c) is incorporated into a drug-delivery platform comprising a biodegradable polymer matrix.

6. The composition of Claim 1, wherein the silk fibroin material is in the form of a silk hydrogel, a silk film, a silk scaffold, a silk sheet, a silk porous material, a silk fiber, or any combination thereof.

7. A composition as defined in Claim 1 for use in a therapeutic method of treating a soft tissue condition.

8. Components (a)-(c) as defined in Claim 1 for use in a therapeutic method of treating a soft tissue condition, the method comprising the steps of administering the adipose tissue to a site of the soft tissue condition, administering the silk fibroin material to the site of the soft tissue condition, and administering compound (c) to the site of the soft tissue condition.

9. Components (a)-(c) for use according to Claim 8, wherein the method comprises administering the silk fibroin material at the same time as administering the compound (c).

10. Composition for use according to Claim 7 or components (a)-(c) for use according to Claim 8, wherein the soft tissue condition is a breast tissue condition, a facial tissue condition, a neck condition, a skin condition, an upper arm condition, a lower arm condition, a hand condition, a shoulder condition, a back condition, a torso condition including an abdominal condition, a buttock condition, an upper leg condition, a lower leg condition including a calf condition, a foot condition including plantar fat pad condition, an eye condition, or a genital condition.

11. Composition for use according to Claim 7 or components (a)-(c) for use according to Claim 8, wherein the soft tissue condition is urinary incontinence, fecal incontinence, or gastroesophageal reflux disease (GERD).

12. A cosmetic method of treating a soft tissue condition, the method comprising administering a composition as defined in any of Claims 1-6 or administering components (a)-(c) as defined in Claim 1 to a site of the soft tissue condition.

## Patentansprüche

1. Zusammensetzung umfassend:
(a) ein Fettgewebe;
(b) ein Seidenfibroinmaterial, das nicht mehr als 1 % (G/G) Sericin umfasst; und
(c) eine Verbindung, dargestellt durch die nachstehende Formel (IX) oder ein pharmazeutisch annehmbares Salz davon: 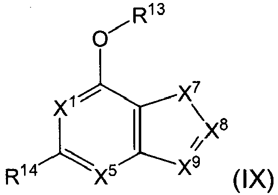 worin:
X¹ und X⁵ unabhängig CH oder N sind;
X⁷ NH, O oder S ist;
X⁸ und X⁹ unabhängig CH oder N sind;
R¹³ gegebenenfalls substituiertes Aryl oder gegebenenfalls substituiertes Heteroaryl ist; und
R¹⁴ C₁₋₆-Alkyl, gegebenenfalls substituiertes Aryl oder gegebenenfalls substituiertes Heteroaryl ist.

2. Zusammensetzung gemäss Anspruch 1, wobei Verbindung (c) eine Verbindung, dargestellt durch die nachstehende Formel (X), oder ein pharmazeutisch annehmbares Salz davon ist: 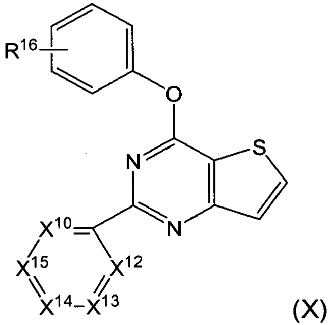 worin:
X¹⁰, X¹², X¹³, X¹⁴ und X¹⁵ unabhängig CR¹⁹ oder N sind;
R¹⁶ unabhängig H, OH, O, F, Cl, Br, I, C₁₋₆-Alkyl, -O-C₁₋₆-Alkyl, CN oder C₁₋₆-Alkyl-CN ist; und
R¹⁹ unabhängig H, OH, O, F, Cl, Br, I, C₁₋₁₀-Alkyl, -O-C₁₋₁₀-Alkyl oder -O-C₄₋₁₀-Alkyl-heterocyclyl ist.

3. Zusammensetzung gemäss Anspruch 1, wobei die Verbindung (c) eine Verbindung, dargestellt durch die nachstehende Formel (XI), oder ein pharmazeutisch annehmbares Salz davon ist: 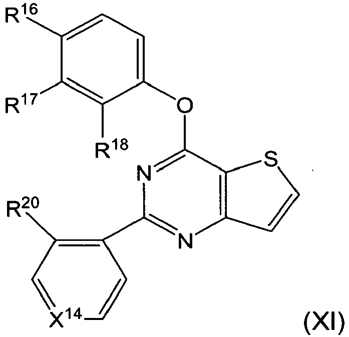 worin:
X¹⁴ CR¹⁹ oder N ist;
R¹⁶, R¹⁷ und R¹⁸ unabhängig H, OH, O, F, Cl, Br, I, C₁₋₆-Alkyl, -O-C₁₋₆-Alkyl, CN oder C₁₋₆-Alkyl-CN sind;
R¹⁹ H, OH, O, F, Cl, Br, I, C₁₋₁₀-Alkyl, -O-C₁₋₁₀-Alkyl oder 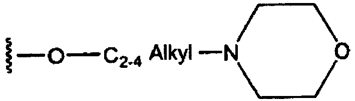 ist; und
R²⁰ H, F, Cl, Br oder I ist.

4. Zusammensetzung gemäss Anspruch 1, wobei die Verbindung (c) eine der nachstehenden Verbindungen oder ein pharmazeutisch annehmbares Salz davon ist:

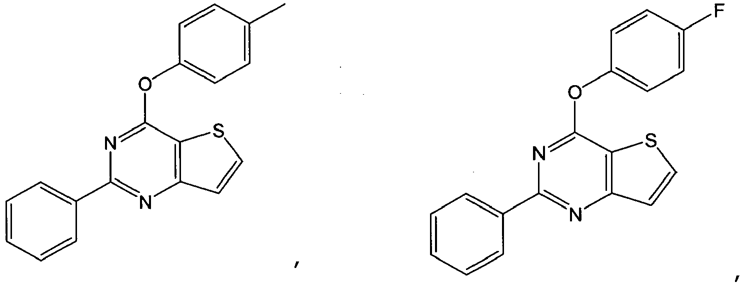

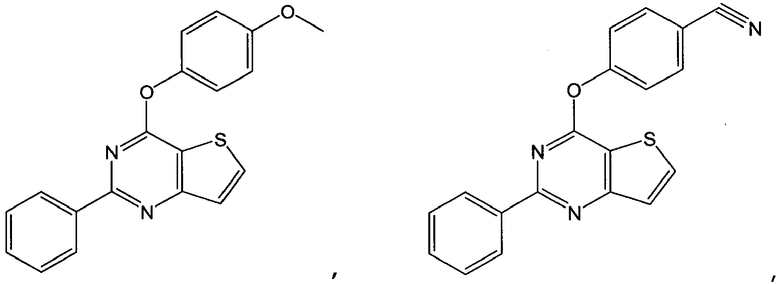

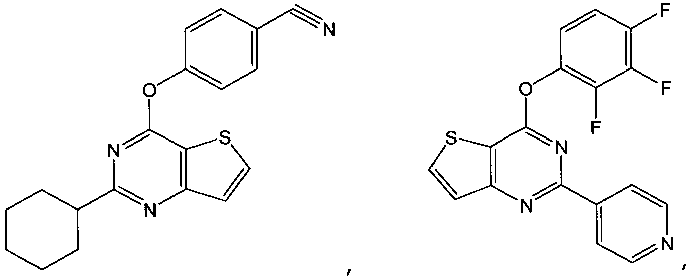

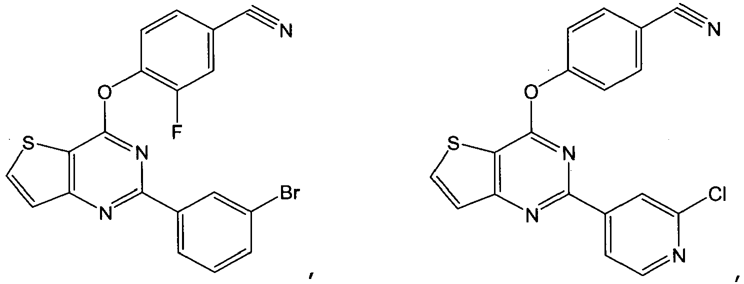

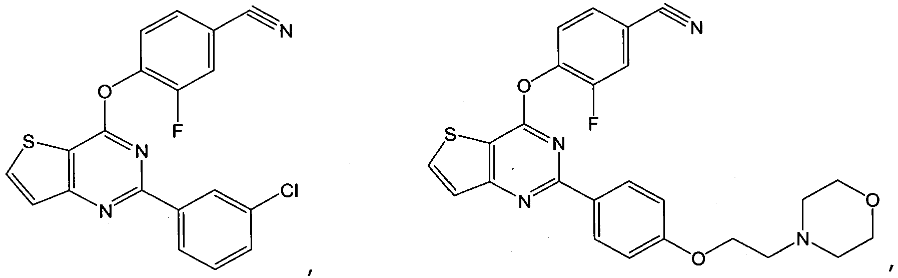

5. Zusammensetzung gemäss Anspruch 1, wobei die Verbindung (c) in eine Arzneimittel-Abgabeplattform integriert ist, die eine biologisch abbaubare Polymermatrix umfasst.

6. Zusammensetzung gemäss Anspruch 1, wobei das Seidenfibroinmaterial in Form eines Seidenhydrogels, eines Seidenfilms, eines Seidengerüsts, eines Seidenblattes, eines porösen Seidenmaterials, einer Seidenfaser oder beliebigen Kombinationen davon vorliegt.

7. Zusammensetzung, wie in Anspruch 1 definiert, zur Verwendung in einem therapeutischen Verfahren zur Behandlung eines Weichgewebeleidens.

8. Komponenten (a) bis (c), wie in Anspruch 1 definiert, zur Verwendung in einem therapeutischen Verfahren zur Behandlung eines Weichgewebeleidens, wobei das Verfahren die Schritte umfasst: Verabreichen des Fettgewebes an die Stelle des Weichgewebeleiden, Verabreichen des Seidenfibroinmaterials an die Stelle des Weichgewebeleidens und Verabreichen der Verbindung (c) an die Stelle des Weichgewebeleidens.

9. Komponenten (a) bis (c) zur Verwendung gemäss Anspruch 8, wobei das Verfahren umfasst: Verabreichen des Seidenfibroinmaterials zur gleichen Zeit wie das Verabreichen der Verbindung (c).

10. Zusammensetzung zur Verwendung gemäss Anspruch 7 oder Komponenten (a) bis (c) zur Verwendung gemäss Anspruch 8, wobei das Weichgewebeleiden ein Brustgewebeleiden, ein Gesichtsgewebeleiden, ein Halsleiden, ein Hautleiden, ein Oberarmleiden, ein Unterarmleiden, ein Handleiden, ein Schulterleiden, ein Rückenleiden, ein Torsoleiden, einschliessich eines abdominalen Leidens, ein Gesässleiden, ein Oberschenkelleiden, ein Unterschenkelleiden, einschliesslich eines Wadenleidens, ein Fussleiden einschliesslich eines Fussohlen-Fettpolsterleidens, ein Augenleiden oder ein Genitalleiden ist.

11. Zusammensetzung zur Verwendung gemäss Anspruch 7 oder Komponenten (a) bis (c) zur Verwendung gemäss Anspruch 8, wobei das Weichgewebeleiden Harninkontinenz, Stuhlinkontinenz oder eine gastroösophageale Refluxkrankheit (GERD) ist.

12. Kosmetisches Verfahren zur Behandlung eines Weichgewebeleidens, wobei das Verfahren umfasst:
Verabreichen einer Zusammensetzung, wie in einem der Ansprüche 1 bis 6 definiert, oder Verabreichen der Komponenten (a) bis (c), wie in Anspruch 1 definiert, an die Stelle des Weichgewebeleidens.

## Revendications

1. Composition comprenant :
(a) un tissu adipeux;
(b) un matériau de fibroïne de soie ne comprenant pas plus de 1 % (M/M) de séricine ; et
(c) un composé représenté par la formule IX suivante ou un sel correspondant, acceptable du point de vue pharmaceutique, 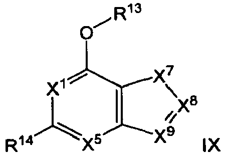 dans laquelle :
X¹ et X⁵ sont indépendamment CH ou N;
X⁷ est NH, O, ou S;
X⁸ et X⁹ sont indépendamment CH ou N ;
R¹³ est un aryle optionnellement substitué ou un hétéroaryle optionnellement substitué ; et
R¹⁴ est un alkyle en C₁₋₆, un aryle optionnellement substitué ou un hétéroaryle optionnellement substitué.

2. Composition selon la revendication 1, dans laquelle le composé (c) est un composé représenté par la formule X suivante ou un sel correspondant, acceptable du point de vue pharmaceutique : 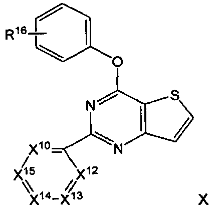 dans laquelle,
X¹⁰, X¹², X¹³, X¹⁴, et X¹⁵ sont les uns indépendamment des autres CR¹⁹ ou N ;
R¹⁶ est indépendamment H, OH, O, F, CI, Br, I, un alkyle en C₁₋₆, un -O-alkyle en C₁₋₆, CN, ou un alkyle en C₁₋₆-CN ; et
R¹⁹ est indépendamment H, OH, O, F, CI, Br, I, un alkyle en C₁₋₁₀, un -O-alkyle en C₁₋₁₀ ou un -O-alkyle en C₄₋₁₀ -hétérocyclyle.

3. Composition selon la revendication 1, dans laquelle le composé (c) est un composé représenté par la formule XI suivante ou un sel correspondant, acceptable du point de vue pharmaceutique : 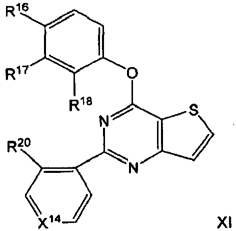 dans laquelle :
X¹⁴ est CR¹⁹ ou N ;
R¹⁶, R¹⁷, et R¹⁸ sont les uns indépendamment des autres H, OH, O, F, Cl, Br, I, un alkyle en C₁₋₆, un-O-alkyle en C₁₋₆, CN, ou un alkyle en C₁₋₆-CN ;
R¹⁹ est H, OH, O, F, Cl, Br, I, un alkyle en C₁₋₁₀, un-O-alkyle en C₁₋₁₀, ou 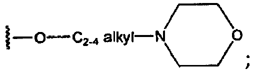 et
R²⁰ est H, F, CI, Br, ou I.

4. Composition selon la revendication 1, dans laquelle le composé (c) est l'un des composés suivants ou un sel correspondant, acceptable du point de vue pharmaceutique :

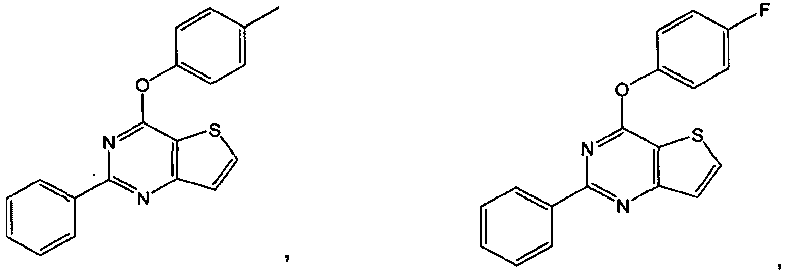

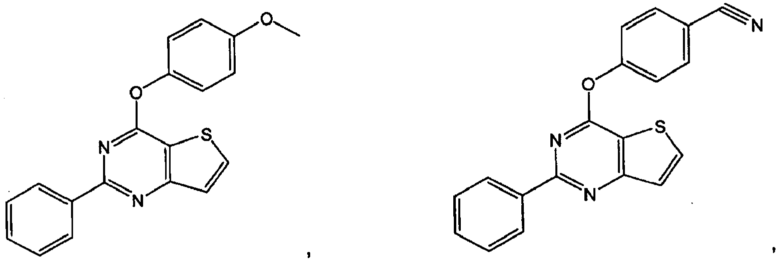

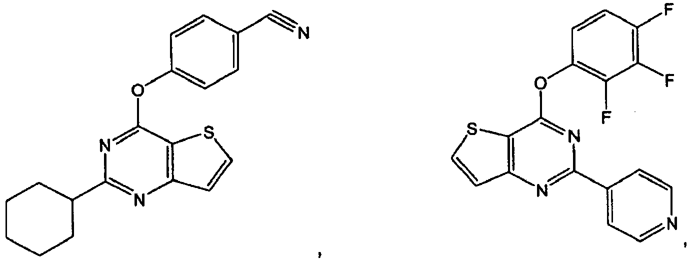

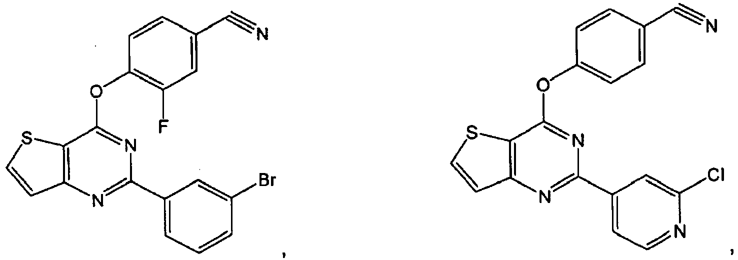

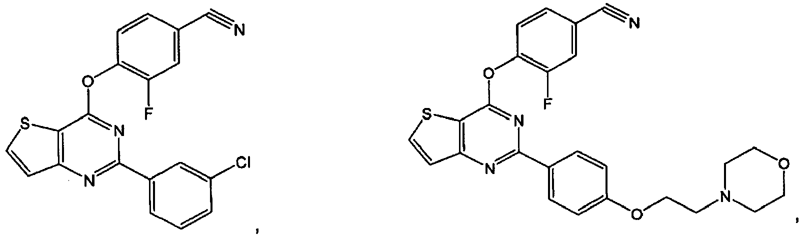

5. Composition selon la revendication 1, dans laquelle le composé (c) est incorporé dans une plate-forme de distribution de médicaments comprenant une matrice polymère biodégradable.

6. Composition selon la revendication 1, dans laquelle le matériau de fibroïne de soie est sous la forme d'un hydrogel de soie, d'une pellicule de soie, d'un échafaudage de soie, d'une feuille de soie, d'un matériau poreux de soie, d'une fibre de soie, ou toute combinaison de ceux-ci.

7. Composition telle que définie dans la revendication 1, destinée à être utilisée dans une méthode thérapeutique pour le traitement d'une pathologie de tissus mous.

8. Composants (a) à (c) tels que définis dans la revendication 1, destinés à être utilisés dans une méthode thérapeutique de traitement d'une pathologie des tissus mous, la méthode comprenant les étapes consistant à administrer le tissu adipeux à un site de la pathologie des tissus mous, administrer le matériau de fibroïne de soie au site de la pathologie des tissus mous, et administrer le composé (c) au site de la pathologie des tissus mous.

9. Composants (a) à (c), destinés à être utilisés selon la revendication 8, où la méthode comprend l'administration du matériau de fibroïne de soie conjointement avec l'administration du composé (c).

10. Composition destinée à être utilisée selon la revendication 7 ou les composants (a) à (c) destinés à être utilisés selon la revendication 8, où la pathologie des tissus mous est une pathologie du tissu mammaire, une pathologie du tissu facial, une pathologie du cou, une pathologie de la peau, une pathologie de la partie supérieure du bras, une pathologie de l'avant-bras, une pathologie de la main, une pathologie de l'épaule, une pathologie du dos, une pathologie du torse incluant une pathologie abdominale, une pathologie de la fesse, une pathologie de la partie supérieure de la jambe, une pathologie de la partie inférieure de la jambe, incluant une pathologie du mollet, une pathologie du pied, incluant la pathologie de la graisse plantaire, une pathologie de l'oeil, ou une pathologie génitale.

11. Composition destinée à être utilisée selon la revendication 7 ou les composants (a) à (c) destinés à être utilisés selon la revendication 8, où la pathologie des tissus mous est l'incontinence urinaire, l'incontinence fécale, la maladie du reflux gastro-oesophagien (RGO).

12. Méthode cosmétique de traitement d'une pathologie des tissus mous, la méthode comprenant l'administration d'une composition telle que définie dans l'une quelconque des revendications 1 à 6, ou l'administration des composants (a) à (c), tels que définis dans la revendication 1, à un site de la pathologie des tissus mous.
